(19) Europäisches Patentamt
European Patent Office
Office européen des brevets

(11) Veröffentlichungsnummer: **0 150 036**
**A1**

(12) # EUROPÄISCHE PATENTANMELDUNG

(21) Anmeldenummer: **85100362.4**

(22) Anmeldetag: **16.01.85**

(51) Int. Cl.[4]: **C 07 D 233/60**
C 07 D 405/12, C 07 D 405/14
C 07 D 249/08, C 07 D 401/12
C 07 D 401/14, C 07 D 409/06
C 07 D 409/12, C 07 D 409/14
A 61 K 31/41, A 61 K 31/415

(30) Priorität: **23.01.84 DE 3402166**

(43) Veröffentlichungstag der Anmeldung:
**31.07.85 Patentblatt 85/31**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Schaper, Wolfgang, Dr.**
**Rauenthaler Weg 18**
**D-6000 Frankfurt am Main 71(DE)**

(72) Erfinder: **Blume, Ernst. Dr.**
**Rossertstrasse 20**
**D-6239 Kriftel(DE)**

(72) Erfinder: **Raether, Wolfgang. Dr.**
**Falkensteinstrasse 6**
**D-6072 Dreieich(DE)**

(72) Erfinder: **Dittmar, Walter. Dr.**
**Uhlandstrasse 10**
**D-6238 Hofheim am Taunus(DE)**

(72) Erfinder: **Alpermann, Hans Georg. Dr.**
**Am Eichkopf 10**
**D-6240 Königstein/Taunus(DE)**

(72) Erfinder: **Sachse, Burkhard. Dr.**
**An der Ziegelei 30**
**D-6233 Kelkheim(Taunus)(DE)**

(72) Erfinder: **Hartz, Peter. Dr.**
**An der Ziegelei 28**
**D-6233 Kelkheim(Taunus)(DE)**

(54) **Azolyl-aryl-alkanol-Derivate, Verfahren zu ihrer Herstellung und ihre Verwendung.**

(57) Azolylderivate der Formel I

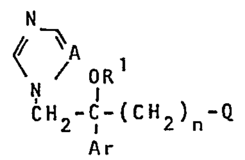

(I)

mit A = CH oder N; n = 2 - 12; Ar = Aromat; $R^1$ = Alkyl, Alkenyl, Alkinyl oder Benzyl; und Q = $-S(O)_{1-3}R^2$ oder $-OR^3$ ($R^2$ = Alkyl, Cycloalkyl, Alkenyl und einige Aromaten; $R^3$ = gesättigtes oder ungesättigtes Alkyl, Cycloalkyl, Cycloalkylalkyl, Aromaten) sowie Verfahren zu deren Herstellung werden beschrieben. Sie sind wirksame antimikrobielle Mittel gegen Pilzinfektionen.

Die Zwischenprodukte der Formel VIIa

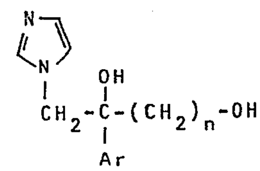

(VIIa)

haben antidepressive Eigenschaften.

EP 0 150 036 A1

Azolyl-aryl-alkanol-Derivate, Verfahren zu ihrer Herstellung und ihre Verwendung

Die Erfindung betrifft neue Azolyl-aryl-alkanol-Derivate, Verfahren zu ihrer Herstellung, neue Ausgangsstoffe hierzu und die Verwendung der neuen Verbindungen.

In den Patentschriften EP-OS 0 054 974 und EP-OS 0 072 623 sind 1-Imidazolyl-2-phenyl-propanol- und 1-Imidazolyl-2-phenyl-octanol-Derivate zur Bekämpfung von Pilzen bei Mensch und Tier beschrieben. Die Wirksamkeit dieser Verbindungen ist jedoch, insbesondere bei niedrigen Konzentrationen, nicht immer ganz befriedigend. Von diesen Verbindungen unterscheiden sich die erfindungsgemäßen Verbindungen wesentlich durch die Art des Alkanol-Restes und durch eine bessere und breitere antimykotische Wirksamkeit.

Im folgenden bedeutet der Ausdruck "Niederalkyl" als solcher oder im Zusammenhang mit anderen Substituenten unverzweigte oder verzweigte Kohlenwasserstoffreste mit 1 bis 6 Kohlenstoffatomen, beispielsweise eine Methyl-, Ethyl-, Propyl-, 1-Methyl-ethyl-, Butyl-, 2-Methyl-propyl-, 1,1-Dimethyl-ethyl-, Pentyl-, 3-Methyl-butyl- oder Hexylgruppe, der Ausdruck "Alkyl" als solcher oder im Zusammenhang mit anderen Substituenten unverzweigte oder verzweigte Alkylreste mit 1-12 Kohlenstoffatomen, wie z.B. die vorstehend genannten Reste oder die Heptyl-, 3-Ethyl-pentyl-, Octyl-, Nonyl-, Deyl-, Undecyl- oder Dodecylgruppe, der Ausdruck "Cycloalkyl" einen Cycloalkylrest mit 3-8 C-Atomen, wie beispielsweise der Cyclopropyl-, Cyclobutyl-, Cyclopentyl-, Cyclohexyl oder

Cyclooctylrest, der Ausdruck "Cycloalkylalkyl" einen mit einer Cycloalkylgruppe substituierten Niederalkylrest, wie z.B. die Cyclopropylmethyl-, Cyclobutylmethyl-, Cyclopentyl-methyl-, Cyclohexylmethyl- oder 2-Cyclohexylethylgruppe,

der Ausdruck "Alkenyl" als solcher oder im Zusammenhang mit anderen Substituenten unverzweigte oder verzweigte Alkenylreste mit 2 bis 10 Kohlenstoffatomen, wie z.B. die 2-Propenyl-, 2-Butenyl-, 3-Methyl-2-butenyl- oder 2-Pentenylgruppe, der Ausdruck "Alkinyl" unverzweigte oder verzweigte Alkinreste mit 2 bis 6-Kohlenstoffatomen wie z.B. die 2-Propinyl-, 2-Butinyl- oder die 2-Pentinylgruppe,

der Ausdruck "Halogenniederalkyl" als solcher oder im Zusammenhang mit anderen Substituenten unverzweigte oder verzweigte Niederalkylreste, bei denen die Wasserstoffatome ganz oder teilweise durch Halogenatome, bevorzugt Fluor oder Chlor ersetzt sind, wie z.B. die Trifluormethyl-, Trichlormethyl-, 1,1,2,2-Tetrafluorethyl- oder die Nonafluorbutylgruppe,

der Ausdruck "Phenylniederalkyl" als solcher oder im Zusammenhang mit anderen Substituenten verzweigte oder bevorzugt unverzweigte Phenylalkylreste mit 1 bis 6 Kohlenstoffatomen im Alkylteil, wie z.B. die Benzyl-, 2-Phenylethyl-, 3-Phenylpropyl-, 4-Phenylbutyl-, 5-Phenylpentyl- oder die 6-Phenylhexylgruppe,

der Ausdruck "Halogen" ein Fluor-, Chlor-, Brom- oder Jodatom.

der Ausdruck "Phenylniederalkenyl" bevorzugt die Cinnamylgruppe.

Gegenstand der Erfindung sind

Azolylalkyl-Derivate der allgemeinen Formel (I)

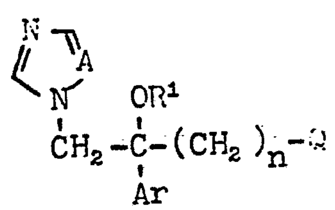

(I)

in welcher

A die CH-Gruppe oder ein Stickstoffatom bedeutet,

n die Zahlen 2 - 12 bedeutet,

Ar eine gegebenenfalls einen, zwei oder drei Substituenten tragende Phenylgruppe bedeutet, wobei die Substituenten gleich oder verschieden sein können und jeweils ein Halogenatom, eine Niederalkyl- oder Niederalkoxygruppe oder eine Trifluormethylgruppe bedeuten, oder auch eine gegebenenfalls mit einem Halogenatom oder einer Niederalkyl- oder Niederalkoxygruppe substituierte Biphenyl-, Naphthyl-, Phenoxyphenyl-, Phenylniederalkyl-, Phenylniederalkoxyphenyl- oder Thienylgruppe bedeutet,

$R^1$ ein Wasserstoffatom, eine Alkyl-, Alkenyl-, Alkinyl- oder eine Benzylgruppe bedeutet, die am Benzolring gegebenenfalls ein oder zwei Substituenten tragen kann, wobei diese gleich oder verschieden sein können und jeweils ein Halogenatom oder eine Niederalkyl-, Niederalkoxy- oder Trifluormethylgruppe bedeuten,

Q entweder

a) einen Rest der allgemeinen Formel (II)

$$-S(O)_x-R^2 \quad (II)$$

bedeutet, wobei x für 0, 1 und 2 steht und $R^2$ eine Alkyl-, Cycloalkyl-, Cycloalkylalkyl-, Alkenyl-, Pyridyl-, 2-Furylmethyl-, Naphthyl-, Halogennaphthyl-, Niederalkylnaphthyl-, Naphthylmethyl-, Phenyl- oder Benzylgruppe bedeutet wobei bei den zwei letztgenannten Gruppen die Benzolringe gegebenenfalls ein oder zwei Substituenten tragen können, die gleich oder verschieden sein können und jeweils Halogen, Niederalkyl, Niederalkoxy, Trifluormethyl, Phenyl, Nitro, Amino oder Aminoacetyl bedeuten können,
oder

b) einen Rest-$OR^3$ bedeutet, wobei
$R^3$ eine Alkyl-, Cycloalkyl-, Cycloalkylalkyl-, Alkenyl-, Alkinyl-, Niederalkoxyniederalkyl-, Niederalkylthioniederalkyl-, Phenylthioniederalkyl-, Phenyloxyniederalkyl-, Thienylmethyl-, Furylmethyl-, Pyridyl-, Naphthyl-, Halogen=naphthyl, Niederalkylnaphthyl-, Niederalkoxy=naphthyl-, Naphthylmethyl-, Phenyl-, Phenylnieder=alkyl- oder Phenylniederalkenylgruppe bedeutet, wobei bei den drei letztgenannten Gruppen die Phenylgruppe gegebenenfalls einen, zwei oder drei Substituenten tragen kann, die gleich oder verschieden sein können und jeweils ein Halogenatom oder eine Niederalkyl-, Halogenniederalkyl-, Nieder=alkoxy-, Halogenniederalkoxy-, Nitro-, Cyano-, Amino-, Aminoacetyl-, Niederalkylamino-, Diniederalkylamino-, Phenyl-, Phenyloxy-, Phenylniederalkyl- oder Phenyl=niederalkoxygruppe bedeuten, mit der Maßgabe, daß nur einer der Substituenten eine Phenyl-, Phenyloxy-, Phenylniederalkyl- oder Phenylniederalkoxygruppe darstellt, die ihrerseits gegebenenfalls einen oder zwei Substituenten tragen können, die gleich oder verschieden sein können und jeweils eine Niederalkyl-, Niederalkoxy-, Nitro-, Cyano- oder Trifluormethylgruppe oder ein Halogenatom bedeuten, und
$R^3$ weiterhin einen Rest der allgemeinen Formel (III) bedeutet

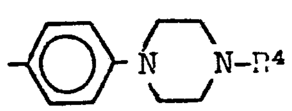 (III)

wobei $R^4$ eine Niederalkyl- oder Niederalkylcarbonylgruppe bedeutet, mit der Maßgabe, daß für diejenigen Verbindungen für die $R^2$ einen Rest der allgemeinen Formel (III) bedeutet, n nicht die Zahl 6 bedeutet, sowie ihre Salze mit Säuren, Metallsalzkomplexe und ihre Stereoisomeren.

Von den Verbindungen der Formel (I) sind solche bevorzugt, für die in dieser Formel Ar eine unsubstituierte oder mit ein oder zwei Halogenatomen, vorzugsweise Fluor, Chlor oder Brom oder mit einer Methyl-, Methoxy- oder Trifluormethylgruppe substituierte Phenylgruppe, eine unsubstituierte oder mit Chlor monosubstituierte Thienylgruppe, eine Biphenyl-, Phenoxyphenyl- oder Naphthylgruppe bedeutet, $R^1$ Wasserstoff, Niederalkyl oder Allyl bedeutet und n eine Zahl zwischen 2 und 8 bedeutet, während Q die vorstehend angegebene Bedeutung hat.

Stärker bevorzugt sind Verbindungen der allgemeinen Formel (I), für die Ar eine mit ein oder zwei Halogenatomen, vorzugsweise Fluor, Chlor oder Brom, substituierte Phenylgruppe oder eine Trifluormethyl-, 4-Methyl- oder 4-Methoxyphenylgruppe oder eine Biphenyl- oder Thienylgruppe bedeutet, n eine Zahl zwischen 2 und 8 bedeutet, $R^1$ ein Wasserstoffatom oder eine Niederalkyl- oder Allylgruppe und Q einen Rest der Formel $SR^2$ und $OR^3$ gemäß der Definition von Q zu Formel I für x gleich null.

Noch stärker bevorzugt sind Verbindungen der allgemeinen Formel (I), für die Ar einen 4-Fluorphenyl-, 4-Chlorphenyl-, 4-Bromphenyl- oder 2,4-Dichlorphenylrest, n eine Zahl zwischen 2 und 8 bedeutet, $R^1$ ein Wasserstoffatom oder eine Niederalkylgruppe bedeutet und Q einen Rest der Formel $SR^2$ oder $OR^3$ wie zu Formel I angegeben, bedeutet und $R^2$ und $R^3$ für Alkyl, Alkenyl, Cycloalkyl, Alkylcycloalkyl, Mono- bzw. Dihalogenbenzyl, Trifluormethylphenyl, Mono- bzw. Dihalogenphenyl, Biphenyl, Naphthyl, Phenoxyphenyl oder 4-Niederalkylpiperazinylphenyl steht.

Am stärksten bevorzugt sind Verbindungen der allgemeinen Formel (I), für die Ar und $R^1$ die im vorstehenden Abschnitt genannten Bedeutungen haben, n eine Zahl zwischen 2 und 5 bedeutet und Q einen Rest der Formel $SR^2$ oder $OR^3$ wie oben angegeben bedeutet, wobei $R^2$ bzw. $R^3$ für Mono- oder Dihalogenphenyl steht, wobei Halogen bevorzugt Fluor, Chlor oder Brom bedeutet oder $R^3$ für Trifluormethylphenyl, Biphenyl oder 4-Niederalkylpiperazinylphenyl steht.

Die Verbindungen der allgemeinen Formel (I) können nach den folgenden Verfahren 1, 2, 3, 4 oder 5 hergestellt werden.

1. Verfahren zur Herstellung von Verbindungen der allgemeinen Formel (Ia) für die Ar, A, n und Q die vorstehend zu Formel (I) angegebenen Bedeutungen haben und $R^{1'}$ Wasserstoff bedeutet,

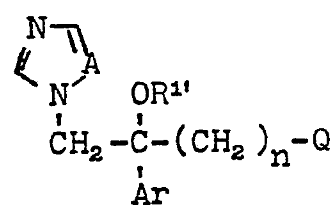

(I a)

dadurch gekennzeichnet, daß man eine Verbindung der allgemeinen Formel (IV)

$$Ar-\underset{\underset{O}{\|}}{C}-(CH_2)_n-Q \qquad (IV)$$

für die Ar, Q und n die vorstehend zu Formel (I) angegebenen Bedeutungen haben,

mit einem Schwefelylid der allgemeinen Formel (A)

$$(CH_3)_2S(O)_zCH_2 \qquad (A) \qquad z = 0,1$$

in Gegenwart eines Verdünnungsmittels zu einer Verbindung der allgemeinen Formel (V)

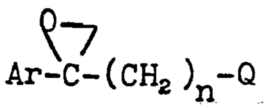 (V)

umsetzt,
für die Ar, n und Q die oben angegebenen Bedeutungen haben,
und anschließend eine Verbindung der allgemeinen Formel (V) mit einer Verbindung der allgemeinen Formel (VI) umsetzt,

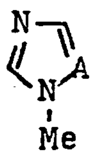 (VI)

in der Me ein Wasserstoffatom oder ein Alkali- oder Erdalkalimetallatom bedeutet, und A die vorstehend zu Formel (I) angegebenen Bedeutungen hat,
und gegebenenfalls

a) eine Verbindung der allgemeinen Formel (I), in der Q $-S-R^2$, wie zu Formel (I) definiert, bedeutet, oxidiert, oder

b) eine Verbindung der allgemeinen Formel (I), in der Q

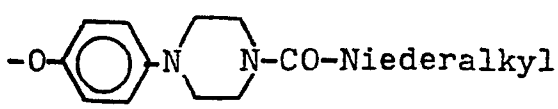 bedeutet,

mittels eines komplexen Metall-Hydrids reduziert.

2. Verfahren zur Herstellung von Verbindungen der allgemeinen Formel (Ib)

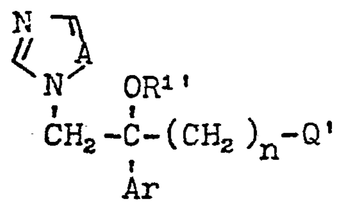 (I b)

worin
A, Ar und n die vorstehend zu Formel (I) angegebenen Bedeutungen haben, $R^{1'}$ Wasserstoff bedeutet und Q' einen Rest OPh oder $S(O)_xPh$ bedeutet, worin x für 0, 1 oder 2 steht und Ph eine Naphthyl-, Halogennaphthyl-, Niederalkylnaphthyl, Niederalkoxynaphthyl- oder eine Phenylgruppe bedeutet, die gegebenenfalls einen, zwei oder drei Substituenten tragen kann, die gleich oder verschieden sein können und jeweils ein Halogenatom oder eine Niederalkyl-, Halogenniederalkyl-, Niederalkoxy-, Halogenniederalkoxy-, Nitro-, Cyano-, Amino-, Aminoacetyl-, Niederalkylamino-, Diniederalkyl=amino-, Phenyl-, Phenyloxy-, Phenylniederalkyl- oder Phenylniederalkoxygruppe bedeuten, mit der Maßgabe, daß nur einer der Substituenten eine Phenyl-, Phenyloxy-, Phenylniederalkyl- oder Phenylniederalkoxygruppe darstellt, die ihrerseits gegebenenfalls einen oder zwei Substituenten tragen kann, die gleich oder verschieden sein können und jeweils eine Niederalkyl-, Niederalkoxy-, Nitro-, Cyano- oder Trifluormethylgruppe oder ein Halogenatom bedeuten,

und für den Fall, daß Q' einen Rest OPh bedeutet, Ph weiterhin einen Rest der allgemeinen Formel (III)

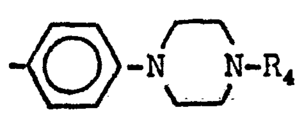 (III)

bedeutet,
wobei $R^4$ eine Niederalkyl- oder Niederalkylcarbonyl-

gruppe bedeutet, mit der Maßgabe, daß für diejenigen Verbindungen, für die $R^3$ einen Rest der allgemeinen Formel (III) bedeutet, n nicht die Zahl 6 bedeutet, dadurch gekennzeichnet, daß man eine Verbindung der allgemeinen Formel (VII)

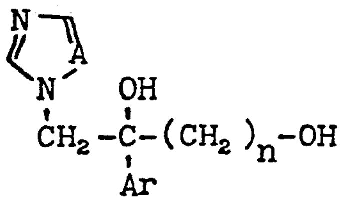
(VII)

in der Ar, A und n die oben angegebenen Bedeutungen haben, mit einer Verbindung der allgemeinen Formel (VIII),

H-XPh (VIII)

in der X Sauerstoff oder Schwefel bedeutet und Ph die oben angegebenen Bedeutungen hat, in Gegenwart von Triphenylphosphin und eines Azodicarbonsäuredialkylesters

Alkyl-OOC-N=N-COO-Alkyl

umsetzt, und die so erhaltenen Verbindungen der Formel (Ib) gegebenenfalls, wie bei Verfahren 1 beschrieben, am Schwefel oxidiert oder an der Amidgruppe reduziert.

3. Verfahren zur Herstellung von Verbindungen der allgemeinen Formel (Ia), dadurch gekennzeichnet, daß man eine Verbindung der allgemeinen Formel (VII),

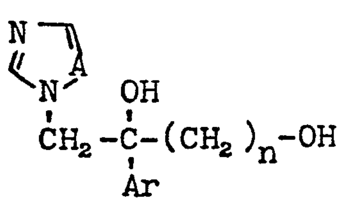
(VII)

in der A, Ar und n die vorstehend zu Formel (I) angegebenen Bedeutungen haben, mit Hilfe eines Halogenierungsmittel oder eines Sulfonylierungsmittels in eine Verbindung der allgemeinen Formel (IX) überführt,

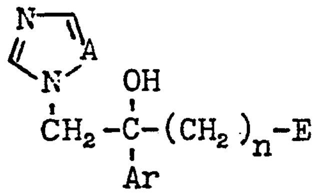

(IX)

in der A, Ar und n die oben angegebenen Bedeutungen haben und E eine reaktive Abgangsgruppe bedeutet, vorzugsweise ein Halogenatom oder eine Alkyl-, Fluoralkyl- oder Arylsulfonyloxygruppe,

und anschließend eine Verbindung der allgemeinen Formel (IX) mit einer Verbindung der allgemeinen Formel (X) umsetzt,

Met-Q" (X)

in der Met ein Alkali- oder Erdalkali-Metallatom bedeutet und Q" einen Rest der allgemeinen Formel $-SR^2$ oder $-OR^3$, worin $R^2$ und $R^3$ die vorstehend zu Formel (I) angegebenen Bedeutungen haben, und gegebenenfalls die so erhaltenen Verbindungen der Formel (Ia), wie bei Verfahren 1 beschrieben, oxidiert oder reduziert.

4. Verfahren zur Herstellung von Verbindungen der allgemeinen Formel (Ic)

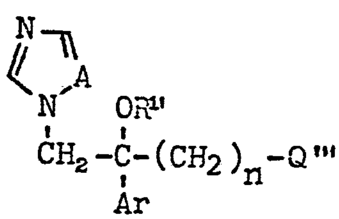

(I c)

in der A, Ar und n die vorstehend zu Formel (I) angegebenen Bedeutungen haben und Q''' einen Rest $OR^{3'}$ bedeutet, worin $R^{3'}$ eine Alkyl-, Cycloalkyl-, Cycloalkylalkyl-, Alkenyl-, Alkinyl-, Niederalkoxyniederalkyl-, Nieder=alkylthioniederalkyl-, Phenylthioniederalkyl-, Phenyl=oxyniederalkyl-, Thienylmethyl-, Furylmethyl-, Naphthylmethyl-, Phenylniederalkyl- oder Phenylnieder=alkenylgruppe bedeutet, wobei bei den zwei letztgenannten Gruppen die Phenylgruppe gegebenenfalls bis zu drei Substituenten tragen kann und diese Substituenten gleich oder verschieden sein können und jeweils ein Halogenatom oder eine Niederalkyl-, Halogenniederalkyl-, Niederalkoxy-, Halogenniederalkoxy-, Nitro-, Cyano-, Amino-, Aminoacetyl-, Niederalkylamino-, Diniederalkylamino-, Phenyl-, Phenyloxy-, Phenylniederalkyl- oder Phenylniederalkoxygruppe bedeuten können, mit der Einschränkung, daß nur einer der Substituenten eine Phenyl-, Phenyloxy-, Phenylniederalkyl- oder Phenylniederalkoxygruppe darstellt, die ihrerseits gegebenenfalls bis zu zwei Substituenten tragen können, wobei diese Substituenten gleich oder verschieden sein können und jeweils eine Niederalkyl-, Niederalkoxy-, Nitro-, Cyano- oder Trifluormethylgruppe oder ein Halogenatom bedeuten,

dadurch gekennzeichnet, daß man eine Verbindung der allgemeinen Formel (VII) in Gegenwart einer starken Base mit einer Verbindung der allgemeinen Formel (XI) umsetzt,

$R^{3'}$-E (XI)

in der $R^{3'}$ die oben angegebenen Bedeutungen hat und E eine reaktive Abgangsgruppe darstellt.

5. Verfahren zur Herstellung von Verbindungen der allgemeinen Formel (Id)

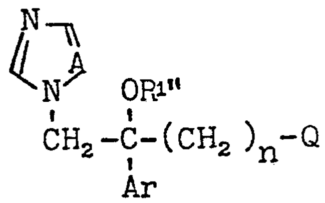 (I d)

in der A, Ar, n und Q die vorstehend zu Formel (I) angegebenen Bedeutungen haben und $R^{1"}$ eine Alkyl-, Alkenyl-, Alkinyl- oder gegebenenfalls wie vorstehend in $R^1$ zu Formel (I) beschriebenen substituierte Benzylgruppe bedeutet, dadurch gekennzeichnet, daß man eine Verbindung der allgemeinen Formel (Ia) mit Hilfe einer starken Base in ein Metallalkoholat überführt und dieses mit einer Verbindung der allgemeinen Formel $ER^{1"}$ umsetzt, in der $R^{1"}$ die oben angegebenen Bedeutungen hat und E eine reaktive Abgangsgruppe mit den oben angegebenen Bedeutungen ist, und die so erhaltenen Verbindungen der Formel (Id) gegebenenfalls, wie bei Verfahren 1 beschrieben, am Schwefel oxidiert oder an einer Amidgruppe reduziert.

ad 1)

Zur Herstellung von Verbindungen der allgemeinen Formel (I a) wird in einem ersten Reaktionsschritt zur Herstellung des Zwischenproduktes (V) ein Keton der Formel (IV),

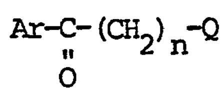 (IV) 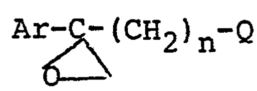 (V)

in der Ar und Q die oben zur Formel (I) angegebenen Bedeutungen haben, mit einem Schwefelylid der allgemeinen Formel (A)

$$(CH_3)_2S(O)_zCH_2 \quad (A) \qquad z = 0,1$$

in einem inerten Lösungsmittel umgesetzt, wobei für z = O ein

Temperaturbereich zwischen - 10 ° und 50 °C, vorzugsweise zwischen 0 und 30 °C zweckmäßig ist, für z = 1 ein Temperaturbereich zwischen 0° und 80°C, vorzugsweise 20 - 50°C, zweckmäßig ist.

Geeignete inerte Lösungsmittel sind hier vorzugsweise Dimethylsulfoxid oder Gemische von Dimethylsulfoxid mit anderen inerten organischen Lösungsmitteln, z.B. Tetrahydrofuran. Die Herstellung der benötigten Schwefel-ylide erfolgt nach in der Literatur beschriebenen Verfahren (vgl. J. Amer. Chem. Soc. 87, 1353 (1965) und J. Amer. Chem. Soc. 84, 3782 (1962)).

Die als Ausgangsstoffe für das Verfahren 1) verwendeten Ketone der Formel (IV) werden nach folgendem Reaktionsschema hergestellt:

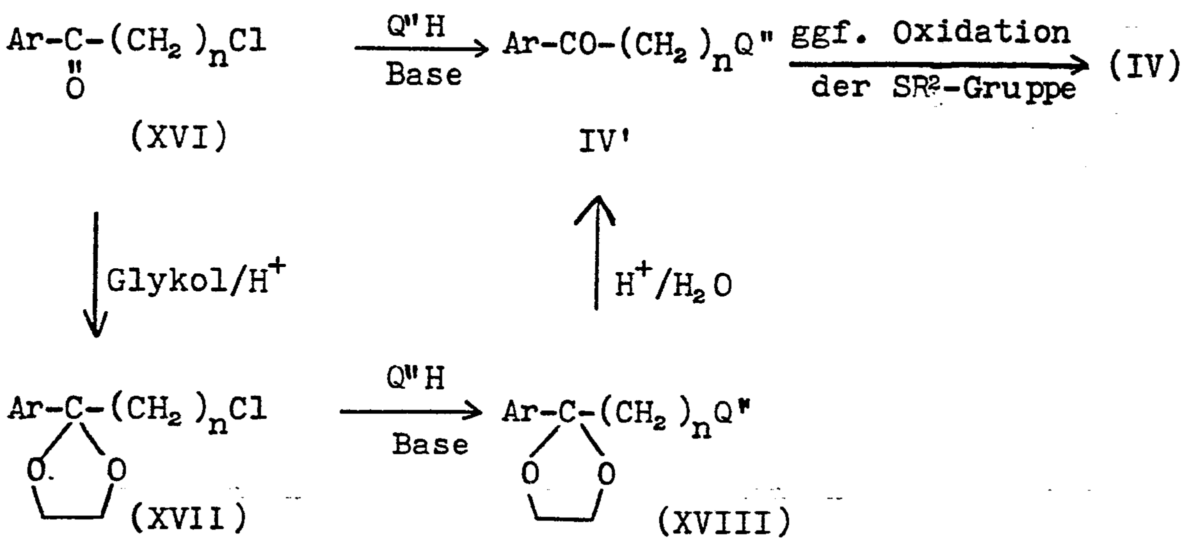


$Q" = SR^2, OR^3$

Die Herstellung von Verbindungen der allgemeinen Formeln (XVI) und (XVII) ist in der Literatur beschrieben (vgl. z.B. Industrie Chimique Belge 9, 1073 (1960), US-Pat. 3 382 250) oder kann in Analogie zu Literaturvorschriften erfolgen.

Die Herstellung der Ether-Derivate (XVIII) und (IV') sowie die Verseifung der Ketale (XVIII) zu den Verbindungen (IV') erfolgen in Analogie zu bekannten Methoden (vgl. z.B. Organikum, VEB Deutscher Verlag der Wissenschaften, Berlin 1977, S. 253; US-Pat. 3 382 250).

Die Oxidation der Verbindungen der allgemeinen Formel (IV'), worin Q" $SR^2$ bedeutet, zu Verbindungen der allgemeinen Formel IV, worin Q -SO-R oder $-SO_2R^2$ bedeutet, erfolgt wie die nachstehend beschriebene Oxidation der Verbindungen der allgemeinen Formel (Ia).

Die Umwandlung der Zwischenprodukte der Formel (V) in die Endprodukte der Formel (I a) erfolgt in einem zweiten Reaktionsschritt durch Umsetzung mit einer Verbindung der allgemeinen Formel (VI),

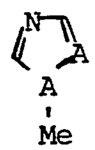 (VI)

in der A die vorstehend angegebene Bedeutung hat und Me ein Wasserstoffatom oder ein Alkali- oder Erdalkalimetallatom, bedeutet. Die Umsetzung kann in Anwesenheit einer starken Base in einem Temperaturbereich von 20 - 150 °C in einem inerten organischen Lösungsmittel erfolgen, wie N,N-Dimethylformamid, N,N-Dimethylacetamid, Dimethylsulfoxid, Sulfolan, N-Methylpyrolidon-2, Dioxan, Tetrahydrofuran, Acetonitril oder einem aromatischen Kohlenwasserstoff wie Benzol, Toluol, Xylol oder Chlorbenzol. Es können auch Gemische dieser Lösungsmittel verwendet werden. Geeignete Basen sind beispielsweise Alkali- oder Erdalkalimetallhydride oder -amide wie z.B. Natriumhydrid oder Natriumamid.

Der zweite Reaktionsschritt kann auch so durchgeführt werden, daß man die Verbindungen der Formel (V) mit Imidazol oder Triazol in äquivalenten Mengen oder auch mit Imidazol oder Triazol im Über-

schuß ohne Lösungsmittel in einem Temperaturbereich von 90 - 160 °C zur Reaktion bringt.

In Zusammenfassung des ersten und zweiten Reaktionsschrittes kann die Umwandlung der Verbindungen der Formel (IV) in solche der Formel (I a) auch ohne Isolierung der Zwischenprodukte (V) als Eintopfverfahren durchgeführt werden. Hierzu stellt man zunächst wie oben beschrieben eine Lösung einer Verbindung der allgemeinen Formel (V) in einem inerten Lösungsmittel her und gibt anschließend zu dieser Lösung eine Verbindung der allgemeinen Formel (VI) und gegebenenfalls ein weiteres Äquivalent einer der oben genannten Basen zu und verfährt weiter wie oben beschrieben.

Gegebenenfalls kann im Anschluß eine Verbindung der Formel (I a), für die Q den Rest $-S-R^2$, wie in Formel I definiert bedeutet, mit einem Oxidationsmittel in einem inerten Lösungsmittel zu einer Verbindung (I a) für die Q den Rest $-\overset{\overset{O}{\|}}{S}-R^2$ oder $-\underset{\underset{O}{\|}}{\overset{\overset{O}{\|}}{S}}-R^2$ bedeutet, oxidiert werden. Geeignete Oxidationsmittel für die Oxidation zum Sulfoxid sind z.B. Wasserstoffperoxid oder Persäuren wie Peressigsäure oder m-Chlorperbenzoesäure oder Natrium- oder Kaliummetaperjodat; für die Oxidation zum Sulfon sind mit Ausnahme der Metaperjodate die gleichen Oxidationsmittel geeignet. Geeignete Lösungsmittel für die Oxidation mit Persäuren sind z.B. Methylenchlorid oder Chloroform, für die Oxidation mit Perjodaten Alkohol/Wasser-Gemische.

Weiterhin kann gegebenenfalls eine Verbindung (I a), für die Q

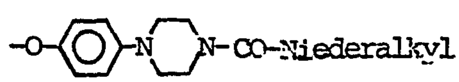

bedeutet, mit einem komplexen Metallhydrid, wie Lithiumaluminiumhydrid oder Diisobutylaluminiumhydrid in einem inerten Lösungsmittel, wie Diethylether, Dimethoxyethan oder Tetrahydrofuran, zum entsprechenden Amin reduziert werden.

ad 2)
Zur Herstellung von Verbindungen der allgemeinen Formel (Ib)

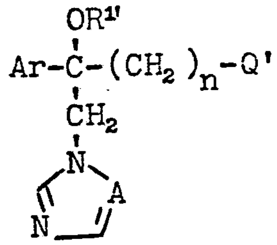

(I b)

worin A, Ar und n die vorstehend zu Formel (Ib) angegebenen Bedeutungen haben, $R^{1'}$ Wasserstoff bedeutet und Q' einen Rest -OPh oder $-S(O)_xPh$ bedeutet, worin x für 0, 1 oder 2 steht und Ph einen aromatischen Rest mit den vorstehend zur Formel (Ib) angegebenen Bedeutungen darstellt, wird eine Verbindung der allgemeinen Formel (VII)

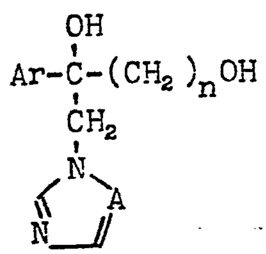

(VII) HXPh (VIII)

in der Ar, A und n die vorstehend angegebenen Bedeutungen haben, mit einem Phenol oder Thiophenol der allgemeinen Formel (VIII), in der X Sauerstoff oder Schwefel bedeutet und PH die oben angegebenen Bedeutungen hat, in Gegenwart von Triphenylphosphin und eines Azodicarbonsäuredialkylesters, bevorzugt Azodicarbonsäuredimethyl- oder -diethylester, in einem geeigneten Lösungsmittel, wie z.B. Tetrahydrofuran, Dioxan, Diethylether, Benzol oder Toluol, umgesetzt (vgl. J.Chem.Soc. Perkin 1, 1975, 461; Helv.Chim. Acta 59 2100 (1976)).

Gegebenenfalls können die so erhaltenen Verbindungen noch wie oben beschrieben am Schwefel der $-SR_2$-Gruppe oxidiert

oder an der Amidgruppe reduziert werden.

Verfahren zur Herstellung der Verbindungen (VII) sind nachstehend beschrieben. Triazol-Derivate, für die n die Zahl 2 bedeutet, können analog dem unter 7. beschriebenen Verfahrens hergestellt werden.

ad 3)
Zur Herstellung von Verbindungen der allgemeinen Formel (Ia) wird in einem ersten Reaktionsschritt eine Verbindung der allgemeinen Formel (VII)

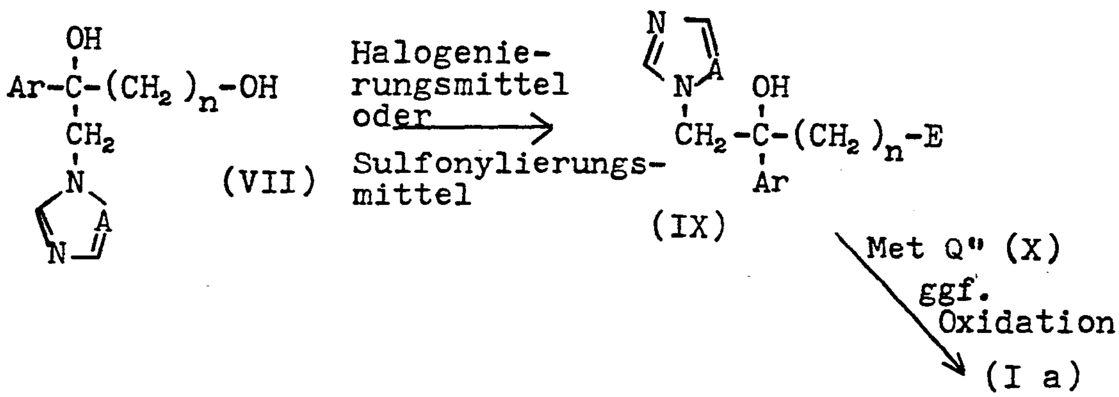


in der Ar, A und n die vorstehend angegebenen Bedeutungen haben, mit Hilfe eines Halogenierungsmittels, wie Thionylchlorid, Phosphortribromid oder Phosphortrijodid oder eines Alkyl-, Halogenalkyl- oder Arylsulfonylhalogenids, wie Methan-, Trifluormethan-, Phenyl-, p-Chlorphenyl- oder p-Toluolsulfonylchlorid, in einem inerten Lösungsmittel, gegebenenfalls in Gegenwart einer Base, in einem Temperaturbereich zwischen 0 und 50 °C in eine Verbindung der allgemeinen Formel (IX) überführt, für

die A, Ar und n die vorstehend angegebenen Bedeutungen haben und E eine reaktive Abgangsgruppe wie Chlor, Brom oder Jod oder eine Sulfonyloxygruppe, z.B. die Methyl-, Trifluormethyl-, Phenyl-, 4-Chlorphenyl- oder 4-Toluol=sulfonyloxygruppe bedeutet. Geeignete Lösungsmittel sind z.B. Methylenchlorid, Chloroform, Acetonitril, Tetrahydroforan, Dioxan, Benzol, Toluol, Pyridin oder auch Wasser oder Gemische der angegebenen Lösungsmittel. Geeignete Basen sind z.B. Alkali- oder Erdalkalimetall-Hydroxide, -Carbonate oder -Hydrogencarbonate, wie Kalium-, Natriumcarbonat oder -Hydrogencarbonat oder Natriumhydroxid oder organische Basen wie z.B. tertiäre Amine wie Triethylamin, Tributylamin oder N-Ethylmorpholin oder Pyridin, Chinolin oder 1H-Imidazol.

In einem zweiten Reaktionsschritt werden Verbindungen der Formel (IX) in einem inerten Lösungsmittel in einem Temperaturbereich zwischen 0 und 150 °C, bevorzugt zwischen 20 und 100 °C, mit einer Verbindung der allgemeinen Formel (X) Met Q" umgesetzt, worin Met ein Alkali- oder Erdalkali-metallatom, bedeutet und Q" einen Rest $-SR_2$ oder $-OR_3$ wie vorstehend angegeben, bedeutet, und die so erhaltenen Verbindungen der Formel (I) gegebenenfalls wie oben beschrieben am Schwefel der $SR_2$-Gruppe oxidiert oder an einer Amidgruppe reduziert. Die Metallsalze (X) werden entweder als solche eingesetzt oder in der Reaktionslösung vor Zugabe der Verbindungen (IX) aus den entsprechenden Verbindungen HQ" mittels geeigneter Basen hergestellt. Solche Basen sind beispielsweise Alkali- oder Erdalkalimetallhydroxide, -alkoholate, -hydride oder -amide wie Natrium- oder Kaliumhydroxid, -methylat, -hydrid oder -amid. Geeignete Lösungsmittel sind z.B. die bei der Herstellung der Verbindungen (Ia) aus den Verbindungen (V) unter "ad 1)" angeführten, sowie Alkohole wie Methanol oder Ethanol oder auch Wasser.

Der zweite Reaktionsschritt kann auch unter den Bedinungen einer Phasentransferreaktion durchgeführt werden, indem man die Reaktionspartner in einem der angegebenen aromatischen Kohlenwasserstoffe unter kräftigem Rühren in Gegenwart eines Phasentransferkatalysators und entweder einem gepulvertem Alkalihydroxid, wie z.B. Natrium- oder Kaliumhydroxid oder einer konzentrierten wäßrigen Lösung derselben, vorzugsweise in einem Temperaturbereich zwischen 20 und 120 °C aufeinander einwirken läßt. Geeignete Phasentransferkatalysatoren sind z.B. Trialkylbenzylammonium- oder Tetraalkylammoniumhalogenide-, hydroxide, -hydrogensulfate mit vorzugsweise 1 - 12 C-Atomen im Alkylrest oder Kronenether, wie z.B. 12-Crown-4, 15-Crown-5, 18-Crown-6 oder Dibenzo-18-crown-6.

ad 4)
Zur Herstellung von Verbindungen der allgemeinen Formel (Ic)

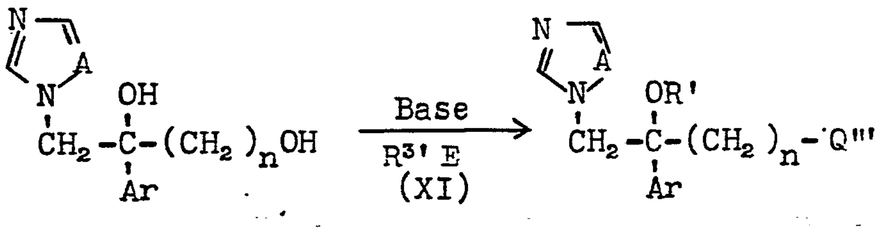


(VII) (I c)

in der Ar, A und n die oben angegebenen Bedeutungen haben, $R^{1'}$ Wasserstoff und Q''' einen Rest $OR^{3'}$ bedeutet, worin $R^{3'}$ die vorstehend zur Formel (Ic) angegebenen Bedeutung besitzt, wird eine Verbindung der allgemeinen Formel (VII) mit

einer Verbindung der allgemeinen Formel (XI ), für die $R^{3'}$ die oben angegebenen Bedeutungen hat und E eine reaktive Abgangsgruppe mit den oben angegebenen Bedeutungen darstellt, in einem inerten Lösungsmittel in Gegenwart von ein bis zwei Äquivalenten einer starken Base in einem Temperaturbereich zwischen 20 und 100 °C umgesetzt. Geeignete Lösungsmittel sind z.B. N,N-Dimethylformamid, N,N-Dimethylacetamid, Dimethylsulfoxid, Sulfolan, Tetrahydrofuran, Dioxan oder Acetonitril. Geeignete Basen sind Erdalkali- und Alkalimetall-hydride und -amide, z.B. Natriumhydrid oder -amid. Die Reaktion wird zweckmäßig so durchgeführt, daß man zunächst mit Hilfe der Base das Dialkoholat der Verbindung (VII) erzeugt und dieses anschließend mit einem Äquivalent der Verbindung (XI) umsetzt.

ad 5) Zur Herstellung von Verbindungen der allgemeinen Formel (I d), in der Ar, A, n und Q die zu Formel (I) angegebenen Bedeutungen haben und $R^{1''}$ eine Alkyl-, Alkenyl-, Alkinyl- oder gegebenenfalls substituierte Benzylgruppe bedeutet,

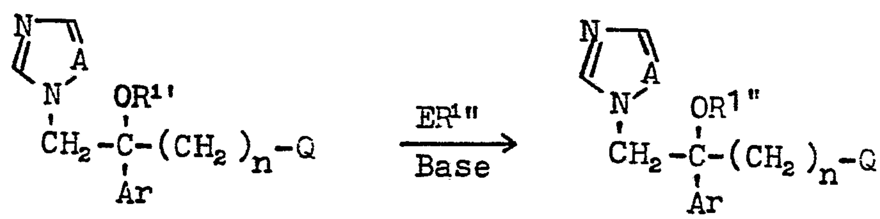


(I a) (I d)

wird eine Verbindung der allgemeinen Formel (I a) mit Hilfe einer starken Base in einem inerten organischen Lösungsmittel in ein Metallalkoholat überführt und dieses mit einer Verbindung der allgemeinen Formel $ER^{1''}$, in der E eine reaktive Abgangsgruppe mit den oben angegebenen Bedeutungen ist und $R^{1''}$ die oben angegebenen Bedeutungen besitzt, in einem Temperaturbereich zwischen 20 und 180 °C, bevorzugt zwischen 40 und 100 °C, umgesetzt. Geeignete Basen sind z.B. Alkali- und Erdalkalimetallhydride- oder Amide wie z.B. Natriumhydrid- oder -amid. Als Lösungsmittel geeignet sind z.B. Dimethylformamid, Dimethylsulfoxid, Sulfolan, Tetrahydrofuran, Acetonitril oder Dioxan. Auch eine Veretherung unter den oben ad 3) beschriebenen Phasentransferbedingungen ist möglich.

Die so erhaltenen Verbindungen der allgemeinen Formel (Id) können gegebenenfalls, wie ad 1) beschrieben, am Schwefel oxidiert oder an der Amidgruppe reduziert werden.

Gegenstand der Erfindung sind weiterhin Imidazolylalkyl-Dialkohole der allgemeinen Formel (VIIa)

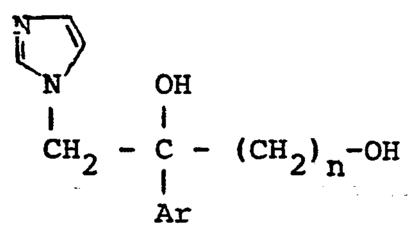

(VIIa)

worin Ar und n die oben zur Formel (I) angegebenen Bedeutungen haben und n bevorzugt eine Zahl zwischen 2 und 5 bedeutet.

Die Verbindungen der allgemeinen Formel (VIIa) können nach den folgenden Verfahren 6 und 7 hergestellt werden:

6. Zur Herstellung der Verbindungen der allgemeinen For-

0150036

mel (VIIa'),

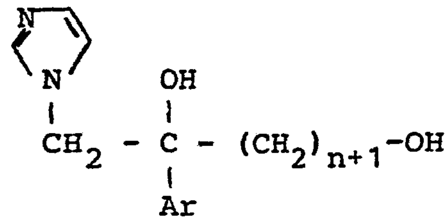

(VIIa')

für die Ar und n die zur Formel (I) angegebenen Bedeutungen haben, wird in einem ersten Reaktionsschritt ein Ketoester der allgemeinen Formel (XII),

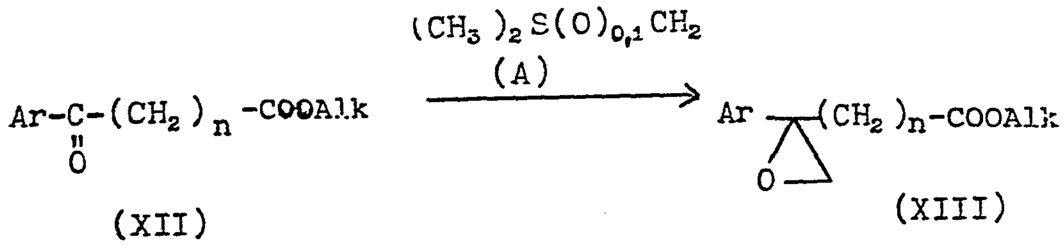


(XII)

(XIII)

worin Ar und n die zur Formel (I) angegebenen Bedeutungen haben und Alk eine Alkylgruppe bedeutet, unter den gleichen Bedingungen, wie bei der Herstellung der Verbindungen der allgemeinen Formel (V) aus solchen der allgemeinen Formel (IV) unter ad 1) beschrieben, mit einem Schwefelylid der allgemeinen Formel (A) zu einer Verbindung der allgemeinen Formel (XIII) umgesetzt, für die Ar, n und Alk die oben angegebenen Bedeutungen haben, und diese Verbindung (XIII) anschließend mit einer Verbindung der allgemeinen Formel (VI') worin Me die zur Formel (VI) angegebenen Bedeutungen hat, umgesetzt, wobei Verbindungen der allgemeinen Formel (XIV) oder auch solche der allgemeinen Formel (XV) entstehen,

(XIII) 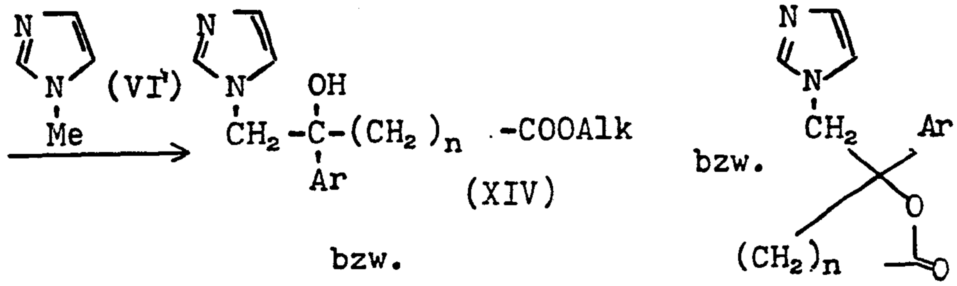


(XIV) bzw. (XV)

bzw.

(XIV) oder (XV) 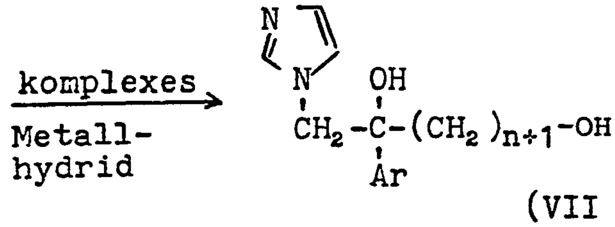


(VII a')

für die Ar, n und Alk die oben angegebenen Bedeutungen haben und aus welchen in einem weiteren Reaktionsschritt durch Reduktion mit einem komplexen Hydrid, z.B. Lithiumaluminiumhydrid, Lithiumborhydrid, Natrium-bis-(2-methoxyethoxy)-aluminiumhydrid oder Diisobutylaluminiumhydrid, in einem inerten Lösungsmittel, z.B. Diethylether, Tetrahydrofuran oder Dimethoxyethan, die gewünschten Verbindungen der allgemeinen Formel (VIIa') erhalten werden.

Die Herstellung von Verbindungen der allgemeinen Formel (XII), bzw. der entsprechenden Ketocarbonsäuren

$$Ar-\overset{O}{\overset{\|}{C}}-(CH_2)_n-COOH,$$

aus denen sie durch Veresterung erhalten werden können, ist beschrieben bzw. kann in Analogie zu Literaturvorschriften erfolgen (vgl. z.B. J.Amer.Chem.Soc. 70, 3197 (1948); Chem.Ber. 107, 210 (1974)).

7. Zur Herstellung von Verbindungen der allgemeinen Formel (VIIa").

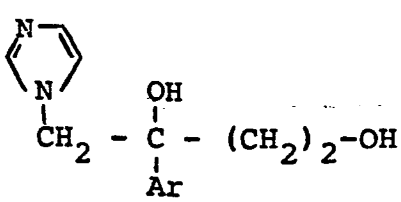

(VIIa")

worin Ar die oben zur Formel (I) angegebenen Bedeutungen hat,
wird in einem ersten Reaktionsschritt ein Imidazolylketon der allgemeinen Formel (XVI),

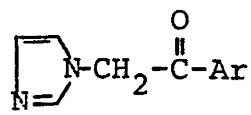
(XVI)

worin Ar die oben angegebenen Bedeutungen hat,
mit einem Essigsäureester $CH_3$-COOAlk, (wobei Alk eine Alkylgruppe bedeutet) in Gegenwart einer Base, z.B. Lithium-bis-(trimethylsilyl)-amid oder Lithium-N-isopropylcyclohexylamid, in einem inerten Lösungsmittel, bevorzugt Tetrahydrofuran, bei tiefen Temperaturen (-78°C bis -40°C) umgesetzt und hierbei eine Verbindung der allgemeinen Formel (XVII) erhalten,

(XVI)
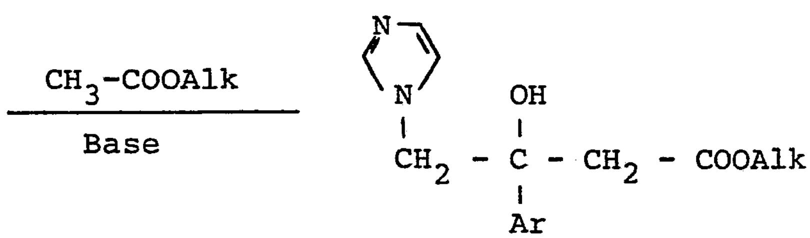

(XVII)

worin Ar und Alk die oben angegebenen Bedeutungen haben.

In einem zweiten Reaktionsschritt werden die Verbindungen der Formel (XVII) mit einem komplexen Metallhydrid, z.B. Lithiumaluminiumhydrid, Diisobutylaluminiumhydrid oder Natrium-bis-(2-methoxyethoxy)-aluminiumhydrid in einem inerten Lösungsmittel, z.B. Diethylether, Tetrahydrofuran oder Dimethoxyethan, zu den Endprodukten (VIIa") reduziert.

Gegenstand der Erfindung sind weiterhin Triazolylalkyl-Dialkohole der allgemeinen Formel (VIIb)

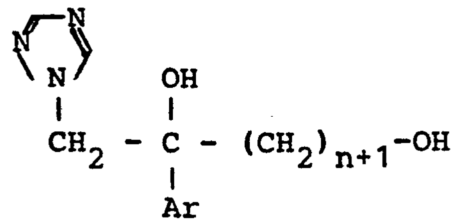

(VIIb)

worin Ar und n die oben zur Formel (I) angegebenen Bedeutungen haben. Sie können nach Verfahren 8 hergestellt werden.

8. Zur Herstellung der Verbindungen der allgemeinen Formel (VIIb) verfährt man, ausgehend von den oben beschriebenen Verbindungen der Formel (XII) analog der unter 6.) beschriebenen Herstellung der Verbindungen der Formel (VIIa'), wobei man anstelle einer der unter 6.) verwendeten Imidazol-Verbindungen der Formel (VI') ein Triazol-Derivat der allgemeinen Formel VII") verwendet,

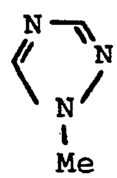

(VII")

worin Me die oben angegebenen Bedeutungen hat, und im übrigen die gleichen Reagentien und Reaktionsbedingungen zur Anwendung kommen läßt.

Die Verbindungen der allgemeinen Formeln (I) und (VIIa) weisen ihre wesentlichen Eigenschaften auch in Form ihrer Salze auf. Zur Herstellung von Säureadditionssalzen kommen alle physiologisch verträglichen Säuren in Frage. Hierzu gehören vorzugsweise die Halogenwasserstoffsäuren, wie z.B. Chlorwasserstoff- und Bromwasserstoff-säure, sowie Salpetersäure, ferner Phosphorsäure oder Schwefelsäure. Bevorzugte organische Säuren sind mono- und bifunktionelle Carbonsäuren und Hydroxycarbonsäuren wie z.B. Essigsäure, Maleinsäure, Oxalsäure, Bernsteinsäure, Fumarsäure, Weinsäure, Zitronensäure, Salicylsäure, Sorbinsäure, Milchsäure sowie Sulfonsäuren wie z.B. p-Toluolsulfonsäure, Methyl- und Phenylsulfonsäure sowie 1,5-Naphthalindisulfonsäure.

Die Salze der Verbindungen der Formeln (I) oder (VIIa) können in einfacher Weise nach ühlichen Salzbildungsmethoden, z.B. durch Lösen einer Verbindung der Formel (I) oder (VIIa) in einem geeigneten inerten Lösungsmittel und Hinzufügen der Säure, z.B. Chlorwasserstoffsäure oder Salpetersäure, erhalten werden und in bekannter Weise, z.B. durch Abfiltrieren, isoliert und gegebenenfalls durch Waschen oder Umkristallisation mit einem inerten organischen Lösungsmittel gereinigt werden.

Aus den Formeln (I) und (VIIa) ist ersichtlich, daß die erfindungsgemäßen Verbindungen ein asymmetrisches Kohlenstoffatom aufweisen, das sich in $\alpha$-Stellung zur Ar-Gruppe befindet. Die bei der Synthese anfallenden Racemate können in üblicher Weise in ihre optischen Antipoden getrennt werden, z.B. durch Umsetzung mit einer optisch aktiven Säure; Trennung der diastereomeren Salze und Freisetzung der reinen Enantiomeren der Verbindungen der Formel (I) oder (VIIa) mittels einer Base.

Die neuen Verbindungen der allgemeinen Formel (I) und ihre Säureadditions-Salze sind wertvolle Arzneimittel. Sie wirken insbesondere antimikrobiell und eignen sich zur Vorbeugung und Behandlung von Pilzinfektionen beim Menschen und bei verschiedenen Säugetierarten.

Die neuen Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C.tropicalis, Torulopsis glabrata und Trichosporon cutaneum oder gegen Protozoen wie Trichomonas vaginalis oder T.fetus, oder

auch gegen gram-positive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als geeignete Anwendungsform der erfindungsgemäßen Verbindung kommen beispielsweise in Frage, Tabletten oder Kapseln, Suspensionen, Lösungen, Gelees, Cremes oder Salben sowie Aerosole in Form von Sprays.

Die Anwendungskonzentration für Lösungen, Gelees, Cremes oder Salben sowie Aerosole in Form von Spray beträgt im allgemeinen zwischen 0,1 - 20 vorzugsweise 0,5 - 5 Gewichtsprozenten.

Die orale Anwendung erfolgt in pharmazeutisch üblichen Zubereitungen, beispielsweise in Form von Tabletten oder Kapseln, die pro Tagesdosis 20 - 500 mg, vorzugsweise 50 bis 300 mg des Wirkstoffes in Mischung mit einem gebräuchlichen Trägerstoff und/oder Konstituents enthalten.

Für die lokale Anwendung können z.B. Suspensionen, Lösungen, Gelees, Cremes, Salben oder Suppositorien verwendet werden.

Für die parenterale Anwendung kommen geeignete Suspensionen oder Lösungen in Frage in einer Anwendungskonzentration zwischen 0,1 - 5 Gewichtsprozenten.

Die als Zwischenprodukte zur Herstellung der allgemeinen Formel (I) benötigten Verbindungen der allgemeinen Formel (VII a) zeigen psychotrope Wirkung. Sie stellen insbesondere gut wirksame Antidepressiva mit einer bisher für dieses Anwendungsgebiet nicht bekannten Struktur dar.

Die neuen Verbindungen der allgemeinen Formel (VIIa) und ihre Säureadditionssalze stellen wertvolle Pharmazeutika dar; sie sind in den für Antidepressiva spezifischen biochemischen und pharmakologischen Testanordnungen gut wirksam. So besitzen sie die Fähigkeit, die an der Maus durch Tetrabenazin induzierte Ptosis zu hemmen. Dieser Test dient als Standardtest auf antidepressive Eigenschaften.

Auch im Test auf Reserpinantagonismus, einer Aufhebung des durch Reserpin verursachten hypothermischen Effektes durch eine antidepressiv wirksame Substanz, zeigen die erfindungsgemäßen Verbindungen außerordentlich günstige Wirkung.

Die neuen Verbindungen sind besonders wertvoll durch eine von den bisher bekannten Antidepressiva abweichende Struktur; bekannten Handelsprodukten sind sie in ihrer Wirkung bei geringerer Toxizität gleichwertig oder überlegen.

Die neuen Verbindungen der allgemeinen Formel (I) sind auch als Biozide wirksam, vorzugsweise die Triazolverbindungen, in denen A Stickstoff bedeutet. Sie zeichnen sich insbesondere durch ihre fungizide Wirksamkeit bei phytopathogenen Pilzen aus. Selbst bereits in das pflanzliche Gewebe eingedrungene pilzliche Krankheitserreger lassen sich erfolgreich bekämpfen. Dies ist besonders

wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt eine Vielzahl verschiedener phytopathogener Pilze, wie z.B. Piricularia oryzae, Plasmopara viticola, verschiedene Rostarten, vor allem aber Venturia inaequalis, Cercospora beticola und Echte Mehltaupilze im Obst-, Gemüse-, Getreide- und Zierpflanzenbau.

Die neuen Verbindungen der allgemeinen Formel (I) eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die neuen Verbindungen können als Spritzpulver, emulgierbare Konzentrate, Versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel z.B. ligninsulfonsaures Natrium, 2,2'-dinaphthylmethan-6,6'-di-sulfonsaures Natrium, dibutylnaphthalin-sulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter

Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid- Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granulierten Inertmaterial. Auch können geeignete Wirkstoffe in der für die Herstellung von Düngemittelgranulaten üblichen Weise - gewünschtenfalls in Mischung mit Düngemitteln, granuliert werden.

In Spritzpulvern beträgt die Wirkstoffkonzentration z.B. etwa 10 - 90 Gew.-%, der Rest zu 100 Gew.-% besteht aus üblichen Formulierungsbestandteilen. Bei emulgierbaren Konzentraten kann die Wirkstoffkonzentration etwa 10 - 80 Gew.-% betragen. Staubförmige Formulierungen enthalten meistens 5 - 20 Gew.-% an Wirkstoff, versprühbare Lösungen etwa 1 - 20 Gew.-%. Bei Granulaten hängt der Wirkstoffgehalt zum Teil davon ab, ob die wirksame Verbindung fküssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z.B. Insektiziden, Akariziden, Herbiziden, Düngemitteln, Wachstumsregulatoren oder weiteren Fungiziden sind gegebenenfalls möglich, wobei u.U. auch synergistische Wirkungssteigerungen erzielt werden können.

Im folgenden seien einige Formulierungsbeispiele angeführt:

Ein Stäubemittel wird erhalten, indem man 10 Gewichtsteile Wirkstoff und 90 Gewichtsteile Talkum als Inertstoff mischt und in einer Schlagmühle zerkleinert.

Ein in Wasser leicht dispergierbares, benetzbares Pulver wird erhalten, indem man 25 Gewichtsteile Wirkstoff, 65 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich

z.B. ca. 255 bis über 377°C) mischt und in einer Reibkugelmühle auf eine Feinheit von unter 5 Mikron vermahlt.

Ein emulgierbares Konzentrat läßt sich herstellen aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

Die nachfolgenden Beispiele dienen zur näheren Erläuterung der Erfindung, ohne dieselbe einzuschränken.

Beispiel 1

2-(4-Chlorphenyl)-6-(4-chlorphenylthio)-1-(1H-imidazol-1-yl)-hexan-2-ol

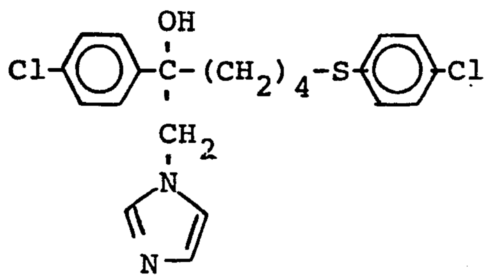

Unter trockener Stickstoffatmosphäre werden 4,3 g Natriumhydrid (80 %-ige Dispersion in Mineralöl) und 33,8 g Trimethylsulfoxoniumjodid vorgelegt und bei 10 - 15 °C Innentemperatur langsam mit 150 ml trockenem Dimethylsulfoxid versetzt. Nach Ende der Wasserstoffentwicklung wird eine Lösung von 43,5 g 1-(4-Chlorbenzoyl)-4-(4-chlorphenylthio)-butan (Beispiel 15) in 200 ml trockenem Dimethylsulfoxid zugetropft. Man rührt 3 Stunden bei 50 °C, kühlt ab und gießt auf Eis. Nach Extraktion mit Methylenchlorid wird mit Wasser gewaschen, getrocknet und eingeengt. Man erhält die Oxiran-Zwischenstufe als ein Öl, das ohne weitere Reinigung mit 50 g Imidazol auf dem Dampfbad 30 Minuten auf 100 °C erhitzt wird. Das Reaktionsgemisch wird mit Wasser/Methylenchlorid aufgenommen, die organische Phase mit Wasser gewaschen, getrocknet und eingeengt.

Beim Verreiben des öligen Rückstands mit Ethylacetat kristallisiert das Produkt aus. Man erhält 39,0 g farblose Kristalle (47,6 % d.Th.).

Fp. 137 - 138 °C

Analyse:

$C_{21}H_{22}Cl_2N_2OS$ MG 421,39

ber.: C 59,86 %; H 5,26 %; N 6,65 %
gef.: C 59,6 %; H 5,2 %; N 6,5 %

Beispiel 2

5-(3,4-Dichlorphenoxy)-2-(4-fluorphenyl)-1-(1,2,4-triazol-1-yl)-pentan-2-ol

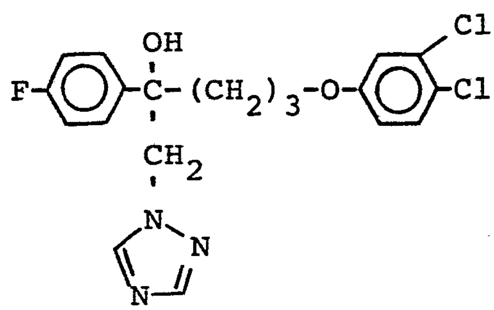

Analog dem vorstehenden Beispiel wird aus 19,0 g Trimethylsulfoxoniumjodid, 4,4 g Natriumhydrid (80 %-ige Dispersion in Mineralöl) und 23,5 g 1-(4-Fluorbenzoyl)-3-(3,4-dichlorphenoxy)-propan (Beispiel 14) in trockenem Dimethylsulfoxid die Oxiran-Zwischenstufe hergestellt. Das Rohprodukt wird mit einem Gemisch aus 10,0 g 1,2,4-Triazol-Natriumsalz und 10,0 g 1,2,4-Triazol in 150 ml trockenem Dimethylformamid 10 Stunden bei Raumtemperatur gerührt. Das Lösungsmittel wird im Vakuum abgezogen, der Rückstand mit Wasser/Methylenchlorid aufgenommen, die organische Phase mit Wasser gewaschen, getrocknet und

eingeengt. Beim Verreiben des öligen Rückstands mit Ethylacetat kristallisieren 14,5 g farblose Kristalle aus (47,4 % d.Th.).

Fp. 121 - 122 °C

Analyse:

$C_{19}H_{18}Cl_2FN_3O_2$ MG 410,28

ber.: C 55,62 %; H 4,42 %; N 10,24 %
gef.: C 55,2 %; H 4,3 %; N 10,1 %

Beispiel 3

2-(4-Chlorphenyl)-7-(3,4-dichlorphenoxy)-1-(1H-imidazol-1-yl)-heptan-2-ol

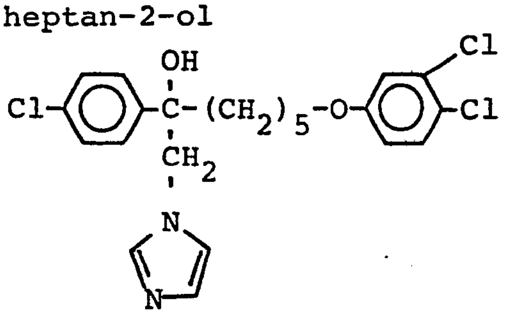

3,1 g 2-(4-Chlorphenyl)-1-(1 H-imidazol-1-yl)-heptan-2,7-diol (Beispiel 13), 1,7 g 3,4-Dichlorphenol und 2,7 g Triphenylphosphin werden in 100 ml trockenem Tetrahydrofuran vorgelegt und 1,9 g Azodicarbonsäurediethylester zugetropft. Man rührt 2 Stunden bei Raumtemperatur, zieht das Lösungsmittel ab und chromatographiert den Rückstand an Kieselgel mit einem Gemisch aus Ethylacetat und Methanol im Verhältnis 19 : 1. Das Produkt wird als letzte Komponente von der Säule eluiert. Man erhält 3,2 g (71 % d.Th.)

eines farblosen Öls das beim Verreiben mit Ethylacetat kristallisiert.

Fp.: 117 - 118 °C

Analyse:

$C_{22}H_{23}Cl_3N_2O_2$ MG: 453,80

ber.: C 58,23 %; H 5,11 %; N 6,17 %
gef.: C 57,9 %; H 5,2 %; N 6,2 %

Beispiel 4

2-(4-Chlorphenyl)-4-(3,4-dichlorphenylthio)-1-(1H-imidazol-1-yl)-butan-2-ol

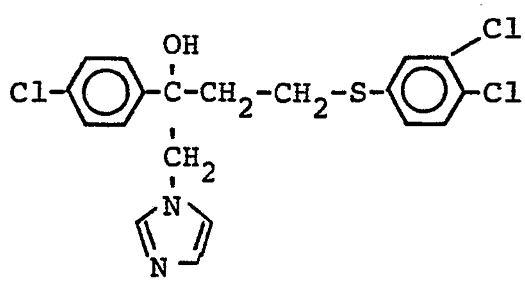

Zu einem Gemisch von 2,7 g 2-(4-Chlorphenyl)-1-(1H-imidazol-1-yl)-butan-2,4-diol (Beispiel 12), 2,0 g 3,4-Dichlorthiophenol und 3,0 g Triphenylphosphin in 60 ml trockenem Tetrahydrofuran tropft man unter Kühlung bei 10 °C eine Lösung von 2,0 g Azodicarbonsäurediethylester in 10 ml trockenem Tetrahydrofuran. Nach Stehen über Nacht wird eingeengt und das Reaktionsgemisch an Kieselgel mit einem Gemisch von Ethylacetat und Methanol im Verhältnis 19 : 1

chromatographiert. Das gewünschte Produkt wird als letzte Komponente von der Säule eluiert. Man erhält ein farbloses Öl, das beim Verreiben mit Diethylether kristallisiert.
Ausbeute: 1,7 g farbl. Kristalle (39,8 % d.Th.)
Fp. 128 - 129 °C.

Analyse:

$C_{19}H_{17}Cl_3N_2OS$ MG 427,78

ber.: C 53,35 %; H 4,01 %; N 6,55 %
gef.: C 53,2 %; H 4,2 %; N 6,6 %

Beispiel 5

2-(4-Chlorphenyl)-1-(1H-imidazol-1-yl)-7-(3-trifluormethyl-phenoxy)-heptan-2-ol

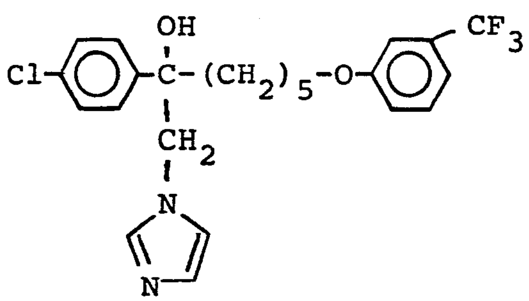

1,6 g 2-(4-Chlorphenyl)-1-(1H-imidazol-1-yl)-heptan-2,7-diol (Beispiel 13) wird in 50 ml Methylenchlorid suspendiert und bei 0 °C eine Lösung von 1,0 g p-Toluolsulfonsäurechlorid in 20 ml Methylenchlorid zugetropft. Man läßt auf Raumtemperatur kommen und rührt 4 Stunden, wobei sich eine klare Lösung bildet. Man zieht das Lösungsmittel ab, gibt 50 ml Toluol, 3 ml 50 %-ige Natronlauge, 0,3 g Tetrabutylammoniumbromid

und 1,0 g 3-Trifluormethylphenol zu und rührt das Gemisch zwei Stunden heftig bei Raumtemperatur und 2 Stunden bei 50 - 60 °C. Nach Abkühlen wird mit Wasser verdünnt, die organische Phase getrocknet und eingeengt. Chromatographie an Kieselgel mit einem Gemisch aus Ethylacetat und Methanol im Verhältnis 9 : 1 ergibt ein farbloses Öl, das beim Verreiben mit Diethylether kristallisiert.
Ausbeute: 0,9 g farblose Kristalle (40 % d.Th.)
Fp. 105 - 107 °C.

Analyse:

$C_{23}H_{24}ClF_3N_2O_2$ MG 452,91

| | | | |
|---|---|---|---|
| ber.: | C 61,00 %; | H 5,34 %; | N 6,19 % |
| gef.: | C 61,0 %; | H 5,5 %; | N 6,1 % |

Beispiel 6

5-(4-Fluorbenzyloxy)-2-(4-fluorphenyl)-1-(1H-imidazol-1-yl)-pentan-2-ol

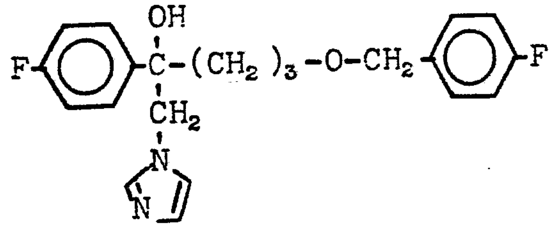

Eine Lösung von 2,6 g 2-(4-Fluorphenyl)-1-(1H-imidazol-1-yl)-pentan-2,5-diol (Beispiel 12) in 50 ml trockenem Dimethylformamid wird mit 1,1 g Natriumhydrid (50 %ige Dispersion in Mineralöl) versetzt und 45 Minuten bei 50 °C gerührt. Man kühlt auf - 20 °C ab und gibt eine Lösung von 1,5 g 4-Fluorbenzylchlorid in 10 ml Dimethylformamid zu. Nach Stehen über Nacht wird das Dimethylformamid abgezogen und der Rückstand mit Wasser/Methylenchlorid aufgearbeitet. Das nach Trocknen und Abziehen des Lösungsmittels erhaltene Produkt wird aus Ethylacetat umkristallisiert. Man erhält 2,0 g farblose Kristalle (54 % d.Th.) Fp. 129 - 130 °C.

Analyse:

$C_{21}H_{22}F_2N_2O_2$ MG: 372,42

ber.: C 67,73 %; H 5,95 %; N 7,52 %
gef.: C 68,3 %; H 6,2 %; N 7,3 %;

Beispiel 7

2-(4-Chlorphenyl)-5-(4-fluorphenoxy)-2-methoxy-1-(1,2,4-triazol-1-yl)-pentan

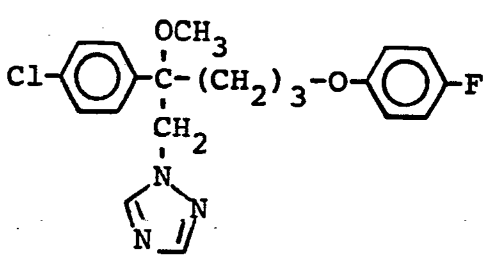

Zu einer Lösung von 5,6 g 2-(4-Chlorphenyl)-5-(4-fluorphenoxy)-1-(1,2,4-triazol-1-yl)-pentan-2-ol (hergestellt analog Beispiel 2) in 50 ml trockenem Dimethylformamid gibt man 0,6 g Natriumhydrid (80 %-ige Dispersion in Mineralöl) und rührt 30 min bei 40 °C. Man kühlt auf

- 20 °C ab und tropft bei dieser Temperatur eine Lösung von 2,8 g Methyljodid in 20 ml Dimethylformamid zu. Man rührt 5 Stunden bei Raumtemperatur nach, zieht das Lösungsmittel am Vakuum ab und nimmt den Rückstand in Wasser/ Methylenchlorid auf. Die organische Phase wird mit Wasser gewaschen, getrocknet und eingeengt. Das Rohprodukt wird durch Chromatographie an Kieselgel mit einem Gemisch aus Methylenchlord/Ethylacetat im Verhältnis 19 : 1 gereinigt. Ausbeute: 4,6 g farbloses Öl (78 % d.Th.)

Analyse:

$C_{20}H_{21}ClFN_3O_2$ MG 389,86

ber.: C 61,62 %; H 5,43 %; N 10,78 %

gef.: C 61,4 %; H 5,1 %; N 10,6 %

Beispiel 8

2-(4-Chlorphenyl)-6-(4-chlorphenylsulfinyl)-1-(1H-imidazol-1-yl)-hexan-2-ol

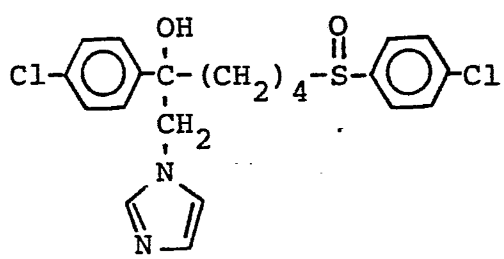

Zu einer Lösung von 15 g 2-(4-Chlorphenyl)-6-(4-chlorphenylthio)-1-(1H-imidazol-1-yl)-hexan-2-ol (Beispiel 1) in 100 ml Methylenchlorid wird bei - 10 °C unter Kühlung eine Lösung von 8,9 g 3-Chlorperbenzoesäure (70 %-ig) in 100 ml Methylenchlorid unter Rühren zugetropft. Man rührt weitere 5 Stunden unter Eiskühlung und schüttelt nach Stehen über Nacht mit 2n Natronlauge und Wasser aus. Nach

Trocknen und Abziehen des Lösungsmittels wird das Rohprodukt an Kieselgel mit einem Gemisch von Ethylacetat und Methanol im Verhältnis 4 : 1 chromatographiert. Ausbeute: 9,6 g amorphes Produkt (61 % d.Th.)

Analyse:

$C_{21}H_{22}Cl_2N_2O_2S$ MG 437,39

ber.: C 57,67 %; H 5,07 %; N 6,40 %
gef.: C 56,7 %; H 5,1 %; N 6,2 %

Beispiel 9

2-(4-Chlorphenyl)-6-(4-chlorphenylsulfonyl)-1-(1H-imidazol-1-yl)-hexan-2-ol

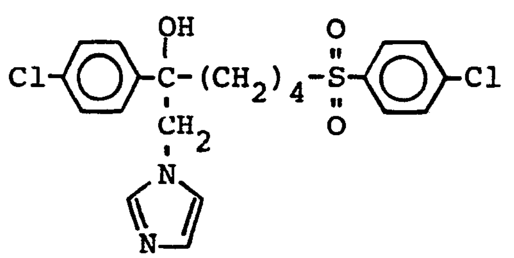

Analog Beispiel 8, aber unter Verwendung einer Lösung von 17,8 g 3-Chlorperbenzoesäure in 150 ml Methylenchlorid erhält man nach Chromatographie an Kieselgel mit Ethylacetat/Methanol 4 : 1 9,2 g farbloses Öl (56 % d.Th.) das beim Verreiben mit Ethylacetat kristallisiert. Fp. 128 - 130 °C

Analyse:

$C_{21}H_{22}Cl_2N_2O_3S$ MG 453,39

ber.: C 55,63 %; H 4,89 %; N 6,18 %
gef.: C 56,0 %; H 4,7 %; N 6,1 %

Beispiel 10

2-(4-Chlorphenyl)-5-[4-(4-ethylpiperazino)-phenoxy]-1-(1,2,4-triazol-1-yl)-pentan-2-ol

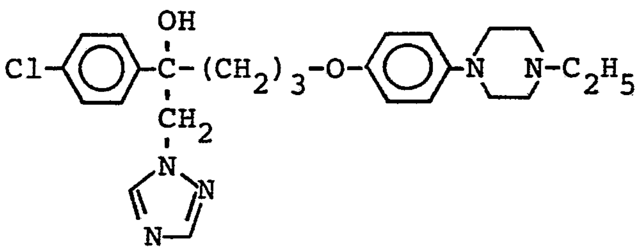

Eine Lösung von 4,0 g 5-[4-(4-Acetyl-piperazino)-phenoxy]-2-(4-chlorphenyl)-1-(1,2,4-triazol-1-yl)-pentan-2-ol (hergestellt analog Beispiel 2) in 30 ml trockenem Tetrahydrofuran tropft man unter Rühren und Kühlung zu einer Lösung von 1,0 g Lithiumaluminiumhydrid in 100 ml trockenem Tetrahydrofuran. Anschließend wird 3 Stunden bei 50 °C nachgerührt, auf 0 °C abgekühlt und mit 10 ml Wasser zersetzt. Nach Stehen über Nacht wird vom anorganischen Material abfiltriert und eingengt. Chromatographie an Kieselgel mit Methanol ergibt 3,2 g farbloses Öl (85 % d.Th.), das beim Stehen durchkristallisiert.
Fp. 131 - 132 °C

Analyse:

$C_{25}H_{32}ClN_5O_2$ MG 470,02

ber.: C 63,89 %; H 6,86 %; N 14,90 %
gef.: C 62,8 %; H 6,7 %; N 14,3 %

Die folgende Tabelle 1 enthält die vorgenannten Verbindungen sowie weitere der allgemeinen Formel (I), die analog zu den angeführten Beispielen oder nach einem der weiter oben geschilderten Verfahren hergestellt werden können.

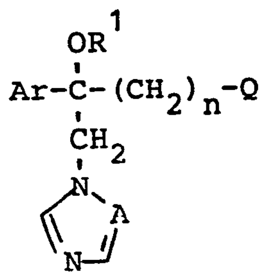

(I)

Tabelle 1

| Verb. Nr. | Ar | A | n | $R_1$ | Q | Fp. /C°7 |
|---|---|---|---|---|---|---|
| 1 | $4-C_6H_4Cl$ | CH | 2 | H | 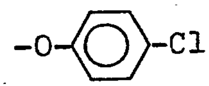 | |
| 2 | $4-C_6H_4Cl$ | CH | 2 | $CH_3$ | 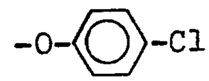 | |
| 3 | $4-C_6H_4Cl$ | CH | 2 | H | 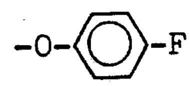 | |
| 4 | $4-C_6H_4Cl$ | CH | 2 | $CH_3$ | 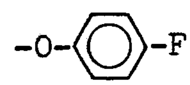 | |
| 5 | $4-C_6H_4Cl$ | CH | 2 | H | 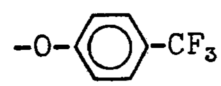 | 162-163 |
| 6 | $4-C_6H_4Cl$ | CH | 2 | $CH_3$ | 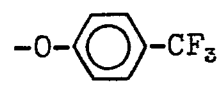 | |
| 7 | $4-C_6H_4Cl$ | CH | 2 | $-CH_2-CH=CH_2$ | 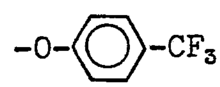 | |
| 8 | $4-C_6H_4Cl$ | CH | 2 | H | 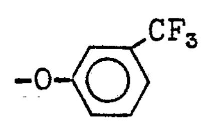 | |
| 9 | $4-C_6H_4Cl$ | CH | 2 | $CH_3$ | 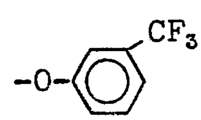 | |
| 10 | $4-C_6H_4Cl$ | CH | 2 | H | 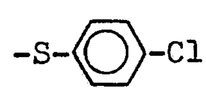 | |
| 11 | $4-C_6H_4Cl$ | CH | 2 | $CH_3$ | 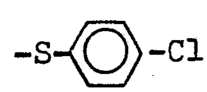 | |

Tabelle 1 (Fortsetzung)

| Verb. Nr. | Ar | A | n | $R_1$ | Q | Fp. [°C] |
|---|---|---|---|---|---|---|
| 12 | $4-C_6H_4Cl$ | CH | 2 | H | 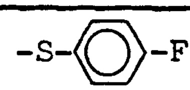 | |
| 13 | $4-C_6H_4Cl$ | CH | 2 | $CH_3$ | 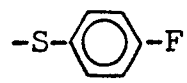 | |
| 14 | $4-C_6H_4Cl$ | CH | 2 | H | 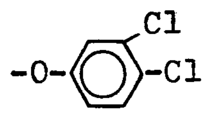 | 139 |
| 15 | $4-C_6H_4Cl$ | CH | 2 | $CH_3$ | 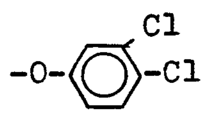 | |
| 16 | $4-C_6H_4Cl$ | CH | 2 | H | 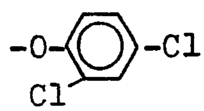 | |
| 17 | $4-C_6H_4Cl$ | CH | 2 | $CH_3$ | 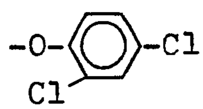 | |
| 18 | $4-C_6H_4Cl$ | CH | 2 | H | 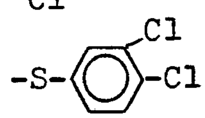 | 128-129 |
| 19 | $4-C_6H_4Cl$ | CH | 2 | $CH_3$ | 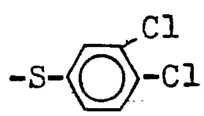 | |
| 20 | $2,4-C_6H_3Cl_2$ | CH | 2 | H | 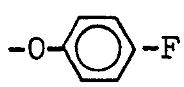 | |
| 21 | $2,4-C_6H_3Cl_2$ | N | 2 | H | 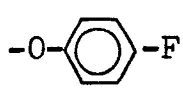 | |
| 22 | $2,4-C_6H_3Cl_2$ | CH | 2 | $CH_3$ | 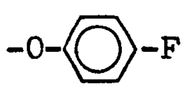 | |

Tabelle 1 (Fortsetzung)

| Verb. Nr. | Ar | A | n | $R_1$ | Q | Fp. [°C] |
|---|---|---|---|---|---|---|
| 23 | 2,4-$C_6H_3Cl_2$ | N | 2 | $CH_3$ | 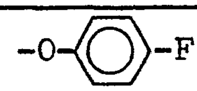 | |
| 24 | 2,4-$C_6H_3Cl_2$ | CH | 2 | H | 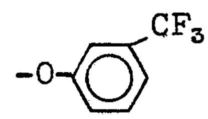 | |
| 25 | 2,4-$C_6H_3Cl_2$ | CH | 2 | $CH_3$ | 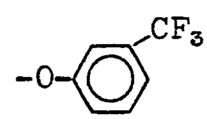 | |
| 26 | 2,4-$C_6H_3Cl_2$ | CH | 2 | H | 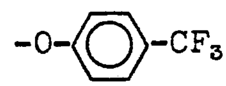 | |
| 27 | 2,4-$C_6H_3Cl_2$ | CH | 2 | $CH_3$ | 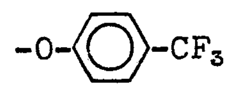 | |
| 28 | 2,4-$C_6H_3Cl_2$ | N | 2 | H | 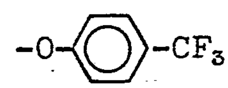 | |
| 29 | 2,4-$C_6H_3Cl_2$ | N | 2 | $CH_3$ | 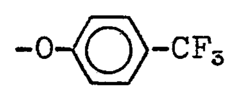 | |
| 30 | 2,4-$C_6H_3Cl_2$ | CH | 2 | H | 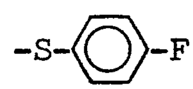 | |
| 31 | 2,4-$C_6H_3Cl_2$ | CH | 2 | $CH_3$ | 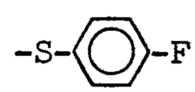 | |
| 32 | 2,4-$C_6H_3Cl_2$ | CH | 2 | H | 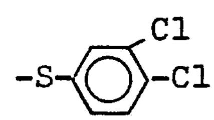 | |
| 33 | 2,4-$C_6H_3Cl_2$ | CH | 2 | $CH_3$ | 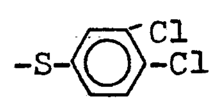 | |

| Verb. Nr. | Ar | A | n | $R_1$ | Q | Fp. [°C] |
|---|---|---|---|---|---|---|
| 34 | $2,4-C_6H_3Cl_2$ | CH | 2 | H | 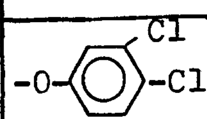 | |
| 35 | $2,4-C_6H_3Cl_2$ | CH | 2 | $CH_3$ | 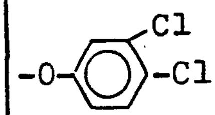 | |
| 36 | $2,4-C_6H_3Cl_2$ | CH | 2 | H | 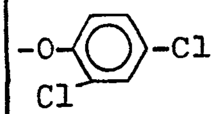 | |
| 37 | $2,4-C_6H_3Cl_2$ | CH | 2 | $CH_3$ | 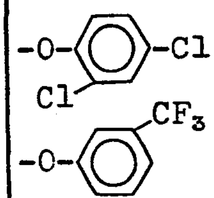 | |
| 38 | $4-C_6H_4F$ | CH | 2 | H | | |
| 39 | $4-C_6H_4F$ | CH | 2 | $CH_3$ | 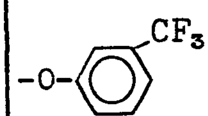 | |
| 40 | $4-C_6H_4F$ | CH | 2 | H | 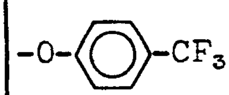 | |
| 41 | $4-C_6H_4F$ | CH | 2 | $CH_3$ | 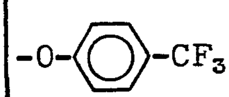 | |
| 42 | $4-C_6H_4F$ | N | 2 | H | 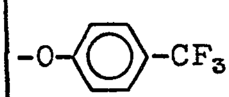 | |
| 43 | $4-C_6H_4F$ | CH | 2 | $CH_3$ | 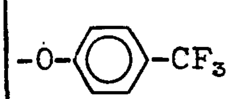 | |
| 44 | $4-C_6H_4F$ | CH | 2 | H | 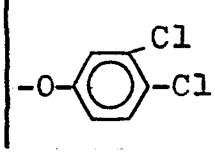 | |

| Verb. Nr. | Ar | A | n | $R_1$ | Q | Fp. /°C/ |
|---|---|---|---|---|---|---|
| 45 | $4-C_6H_4F$ | CH | 2 | $CH_3$ | 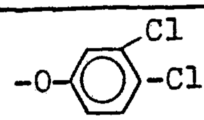 | |
| 46 | $4-C_6H_4F$ | CH | 2 | H | 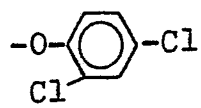 | |
| 47 | $4-C_6H_4F$ | CH | 2 | $CH_3$ | 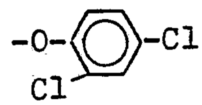 | |
| 48 | $4-C_6H_4F$ | CH | 2 | H | 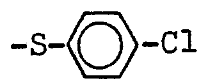 | |
| 49 | $4-C_6H_4F$ | CH | 2 | $CH_3$ | 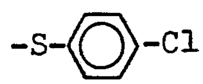 | |
| 50 | $4-C_6H_4F$ | CH | 2 | H | 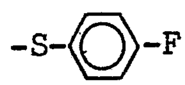 | |
| 51 | $4-C_6H_4F$ | CH | 2 | $CH_3$ | 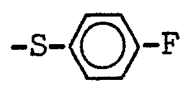 | |
| 52 | $4-C_6H_4F$ | CH | 2 | H | 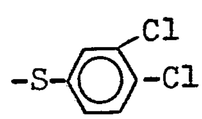 | |
| 53 | $4-C_6H_4F$ | CH | 2 | $CH_3$ | 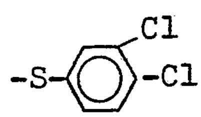 | |
| 54 | $4-C_6H_4Cl$ | CH | 3 | H | $-O-CH_3$ | 109-110 |
| 56 | $4-C_6H_4Cl$ | CH | 3 | 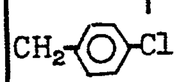 | $-O-CH_3$ | Harz |
| 57 | $4-C_6H_4Cl$ | N | 3 | H | $-O-CH_3$ | 82-83 |

| Verb. Nr. | Ar | A | n | $R_1$ | Q | Fp. [°C] |
|---|---|---|---|---|---|---|
| 58 | 4-$C_6H_3Cl$ | N | 3 | H | $-O-iC_3H_7$ | |
| 59 | 4-$C_6H_4Cl$ | N | 3 | H | $-O-nC_4H_9$ | |
| 60 | 4-$C_6H_4Cl$ | CH | 3 | H | $-O-nC_8H_{17}$ | 103 |
| 61 | 4-$C_6H_4Cl$ | CH | 3 | $CH_3$ | $-O-nC_8H_{17}$ | Harz |
| 62 | 4-$C_6H_4Cl$ | N | 3 | H | $SCH_3$ | |
| 63 | 4-$C_6H_4Cl$ | N | 3 | H | $-S-nC_4H_9$ | |
| 64 | 4-$C_6H_4Cl$ | CH | 3 | H | $-S-tC_4H_9$ | 157-158 |
| 65 | 4-$C_6H_4Cl$ | CH | 3 | H | $-S-nC_8H_{17}$ | 98- 99 |
| 66 | 4-$C_6H_4Cl$ | N | 3 | H | $-S-nC_8H_{17}$ | 75- 76 |
| 67 | 4-$C_6H_4Cl$ | CH | 3 | H | -O- 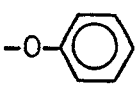 | |
| 68 | 4-$C_6H_4Cl$ | CH | 3 | H | -O- 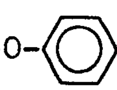 -Cl | 157 |
| 69 | 4-$C_6H_4Cl$ | CH | 3 | $CH_3$ | -O- 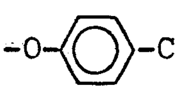 -Cl | Harz |
| 70 | 4-$C_6H_4Cl$ | CH | 3 | $CH_2$ 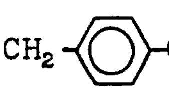 -Cl | -O- 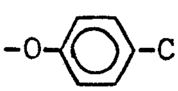 -Cl | Harz |
| 71 | 4-$C_6H_4Cl$ | N | 3 | H | -O- 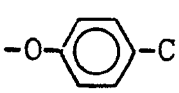 -Cl | |
| 72 | 4-$C_6H_4Cl$ | CH | 3 | H | -O- 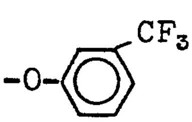 | 112-113 |

| Verb. Nr. | Ar | A | n | $R_1$ | Q | Fp. [°C] |
|---|---|---|---|---|---|---|
| 73 | 4-$C_6H_4Cl$ | CH | 3 | H | 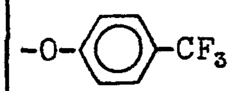 | |
| 74 | 4-$C_6H_4Cl$ | CH | 3 | H | 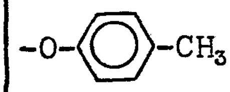 | |
| 75 | 4-$C_6H_4Cl$ | CH | 3 | H | 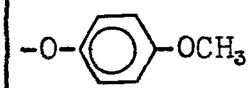 | 114-115 |
| 76 | 4-$C_6H_4Cl$ | N | 3 | H | 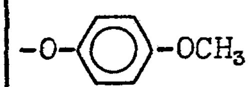 | 87- 88 |
| 77 | 4-$C_6H_4Cl$ | CH | 3 | H | 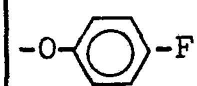 | 152 |
| 78 | 4-$C_6H_4Cl$ | CH | 3 | $CH_3$ | 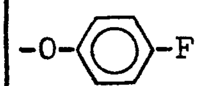 | Harz |
| 79 | 4-$C_6H_4Cl$ | CH | 3 | $CH_2-CH=CH_2$ | 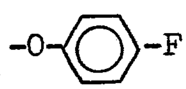 | |
| 80 | 4-$C_6H_4Cl$ | N | 3 | H | 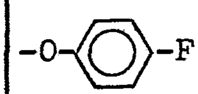 | 127-128 |
| 81 | 4-$C_6H_4Cl$ | N | 3 | $CH_3$ | 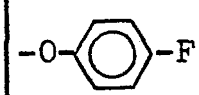 | Harz |
| 82 | 4-$C_6H_4Cl$ | CH | 3 | H | 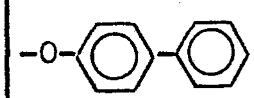 | 188 |
| 83 | 4-$C_6H_4Cl$ | CH | 3 | $CH_3$ | 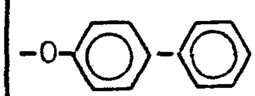 | Harz |
| 84 | 4-$C_6H_4Cl$ | N | 3 | H | 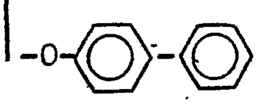 | 153-154 |

| Verb. Nr. | Ar | A | n | $R_1$ | Q | Fp. [°C] |
|---|---|---|---|---|---|---|
| 85 | 4-$C_6H_4F$ | CH | 3 | H | 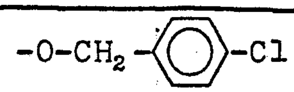 | 138-140 |
| 86 | 4-$C_6H_4F$ | CH | 3 | $CH_3$ | 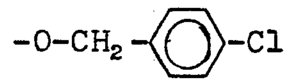 | Oel |
| 87 | 4-$C_6H_4Cl$ | CH | 3 | H | 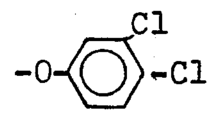 | 126-127 |
| 88 | 4-$C_6H_4Cl$ | CH | 3 | $CH_3$ | 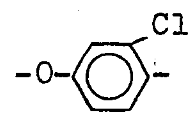 | Oel |
| 89 | 4-$C_6H_4Cl$ | N | 3 | H | 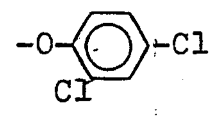 | 126-127 |
| 90 | 4-$C_6H_4Cl$ | CH | 3 | H | 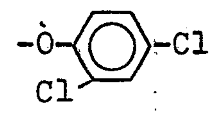 | 127-129 |
| 91 | 4-$C_6H_4Cl$ | CH | 3 | H | 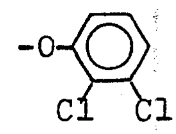 | 154-156 |
| 92 | 4-$C_6H_4Cl$ | N | 3 | H | 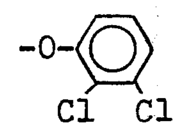 | 102-103 |
| 93 | 4-$C_6H_4Cl$ | CH | 3 | H | 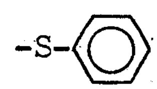 | |
| 94 | 4-$C_6H_4Cl$ | N | 3 | H | 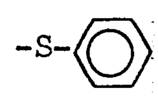 | |
| 95 | 4-$C_6H_4Cl$ | CH | 3 | H | 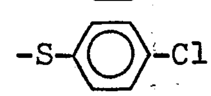 | 112-113 |
| 96 | 4-$C_6H_4Cl$ | CH | 3 | $CH_3$ | 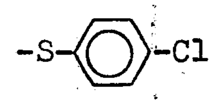 | |

| Verb. Nr. | Ar | A | n | $R_1$ | Q | Fp. [°C] |
|---|---|---|---|---|---|---|
| 97 | $4-C_6H_4Cl$ | N | 3 | H | 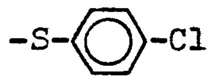 | 78 |
| 98 | $4-C_6H_4Cl$ | CH | 3 | H | 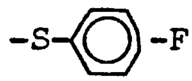 | 120-121 |
| 99 | $4-C_6H_4Cl$ | CH | 3 | $CH_3$ | 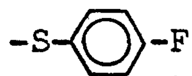 | Harz |
| 100 | $4-C_6H_4Cl$ | CH | 3 | H | 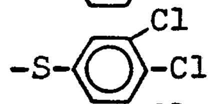 | 108-110 |
| 101 | $4-C_6H_4Cl$ | CH | 3 | $CH_3$ | 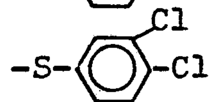 | Harz |
| 102 | $4-C_6H_4Cl$ | N | 3 | H | 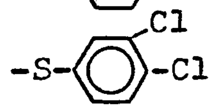 | 117-118 |
| 103 | $4-C_6H_4Cl$ | CH | 3 | H | 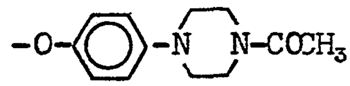 | amorph. |
| 104 | $4-C_6H_4Cl$ | CH | 3 | $CH_3$ | 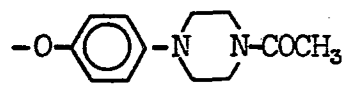 | Harz |
| 105 | $4-C_6H_4Cl$ | N | 3 | H | 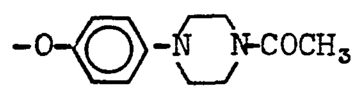 | amorph. |
| 106 | $4-C_6H_4Cl$ | N | 3 | $CH_3$ | 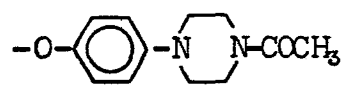 | 123-126 |
| 107 | $4-C_6H_4Cl$ | CH | 3 | H | 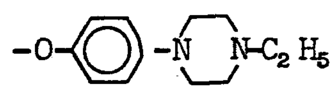 | |
| 108 | $4-C_6H_4Cl$ | N | 3 | H | 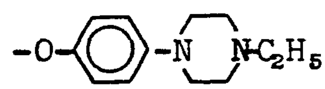 | 131-132 |
| 109 | $4-C_6H_4Cl$ | CH | 3 | H | 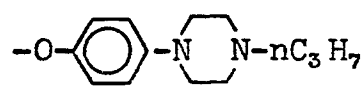 | |
| 110 | $4-C_6H_4Cl$ | N | 3 | H | 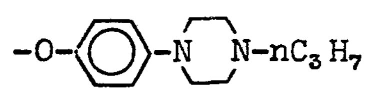 | |
| 111 | $4-C_6H_4Cl$ | CH | 3 | H | 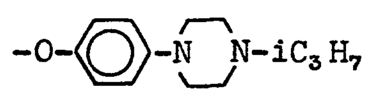 | |

| Verb. Nr. | Ar | A | n | $R_1$ | Q | Fp. [°C] |
|---|---|---|---|---|---|---|
| 112 | $4-C_6H_4Cl$ | N | 3 | H | 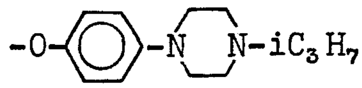 | |
| 113 | $4-C_6H_4Cl$ | CH | 3 | H | 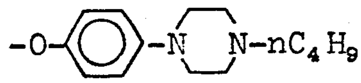 | |
| 114 | $4-C_6H_4Cl$ | N | 3 | H | 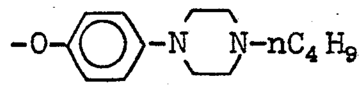 | |
| 115 | $4-C_6H_4Cl$ | CH | 3 | H | 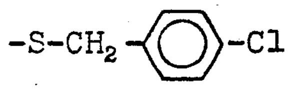 | 135-136 |
| 116 | $4-C_6H_4Cl$ | N | 3 | H | 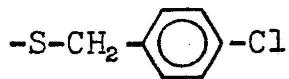 | 98-100 |
| 117 | $4-C_6H_4Cl$ | N | 3 | H | 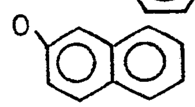 | |
| 118 | $4-C_6H_4F$ | CH | 3 | H | 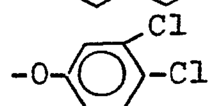 | 153-155 |
| 119 | $4-C_6H_4F$ | N | 3 | H | 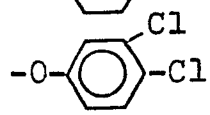 | 121-122 |
| 120 | $4-C_6H_4F$ | CH | 3 | H | 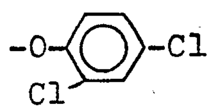 | 160-161 |
| 121 | $4-C_6H_4F$ | N | 3 | H | 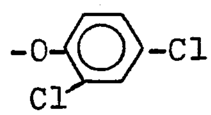 | 116-118 |
| 122 | $4-C_6H_4F$ | CH | 3 | H | 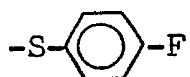 | |
| 123 | $4-C_6H_4F$ | CH | 3 | H | 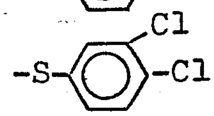 | 149-150 |
| 124 | $4-C_6H_4F$ | CH | 3 | H | 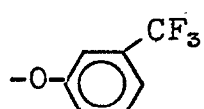 | 125-127 |
| 125 | $4-C_6H_4F$ | N | 3 | H | 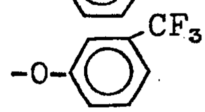 | 88-90 |

| Verb. Nr. | Ar | A | n | $R_1$ | Q | Fp. [°C] |
|---|---|---|---|---|---|---|
| 126 | $4-C_6H_4F$ | N | 3 | H | 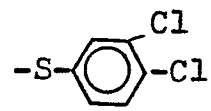 | 82- 84 |
| 127 | $4-C_6H_4F$ | CH | 3 | H | 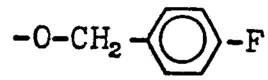 | 129-130 |
| 128 | 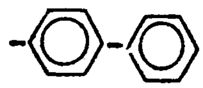 | CH | 3 | H | 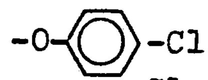 | |
| 129 | 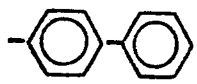 | CH | 3 | H | 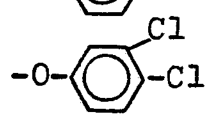 | |
| 130 | 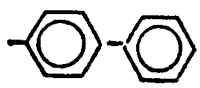 | CH | 3 | H | 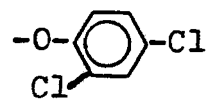 | |
| 130a | 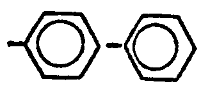 | CH | 3 | H | 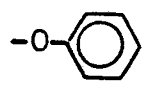 | |
| 131 | 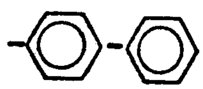 | CH | 3 | H | 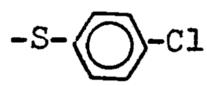 | 163-164 |
| 132 | 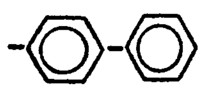 | CH | 3 | $CH_3$ | 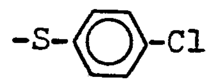 | |
| 133 | 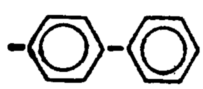 | N | 3 | H | 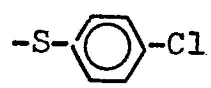 | 160-161 |
| 134 | 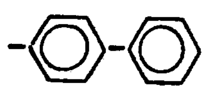 | CH | 3 | H | 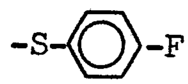 | |
| 135 | 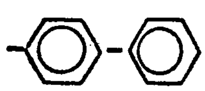 | CH | 3 | H | 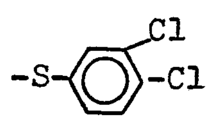 | |
| 136 | 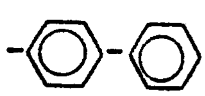 | CH | 3 | H | 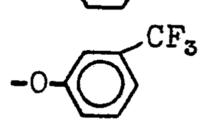 | |
| 137 | 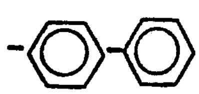 | CH | 3 | H | 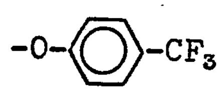 | |
| 138 | 2-Thienyl | CH | 3 | H | 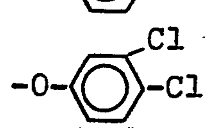 | |

| Verb. Nr. | Ar | A | n | $R_1$ | Q | Fp. [°C] |
|---|---|---|---|---|---|---|
| 139 | 2-Thienyl | CH | 3 | H | 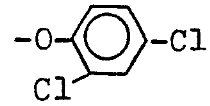 | |
| 140 | " | CH | 3 | H | 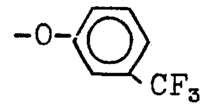 | |
| 141 | " | CH | 3 | H | 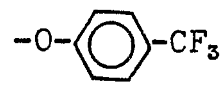 | |
| 142 | " | CH | 3 | H | 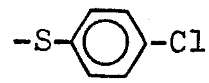 | |
| 143 | " | CH | 3 | H | 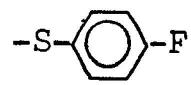 | |
| 144 | " | CH | 3 | H | 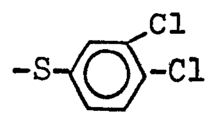 | 146-148 |
| 145 | " | CH | 3 | H | 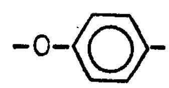 | |
| 146 | 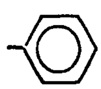 | CH | 3 | H | 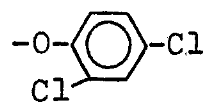 | |
| 147 | 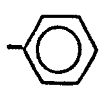 | N | 3 | H | 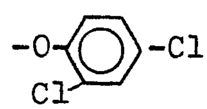 | |
| 148 | 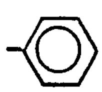 | CH | 3 | H | 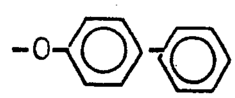 | 170-171 |
| 149 | 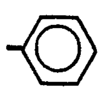 | N | 3 | H | 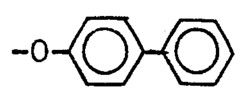 | |
| 150 | 2,4-$C_6H_3Cl_2$ | CH | 3 | H | 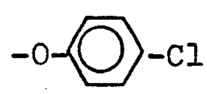 | 145-146 |
| 151 | 2,4-$C_6H_3Cl_2$ | CH | 3 | H | 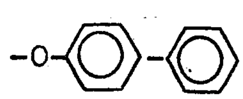 | |

| Verb. Nr. | Ar | A | n | $R_1$ | Q | Fp. [°C] |
|---|---|---|---|---|---|---|
| 152 | 2,4-$C_6H_3Cl_2$ | CH | 3 | H | 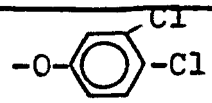 | |
| 153 | 2,4-$C_6H_3Cl_2$ | CH | 3 | H | 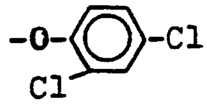 | |
| 154 | 2,4-$C_6H_3Cl_2$ | CH | 3 | H | 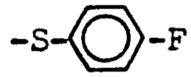 | |
| 155 | 2,4-$C_6H_3Cl_2$ | N | 3 | H | 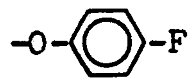 | |
| 156 | 2,4-$C_6H_3Cl_2$ | CH | 3 | H | 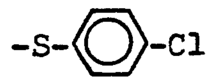 | |
| 157 | 2,4-$C_6H_3Cl_2$ | CH | 3 | H | 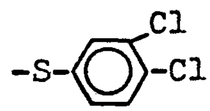 | |
| 158 | 2,4-$C_6H_3Cl_2$ | CH | 3 | H | 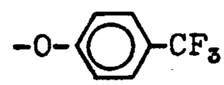 | |
| 159 | 2,4-$C_6H_3Cl_2$ | CH | 3 | H | 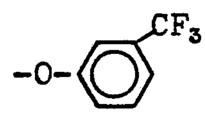 | 126 |
| 160 | 2,4-$C_6H_3Cl_2$ | N | 3 | H | 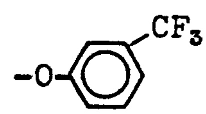 | Harz |
| 161 | 2,4-$C_6H_3Cl_2$ | CH | 3 | H | 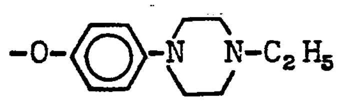 | |
| 162 | 2,4-$C_6H_3Cl_2$ | CH | 3 | H | 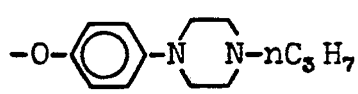 | |
| 163 | 2,4-$C_6H_3Cl_2$ | CH | 3 | H | 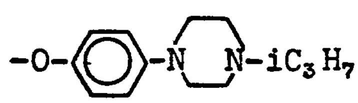 | |
| 164 | 2,4-$C_6H_3Cl_2$ | CH | 3 | H | 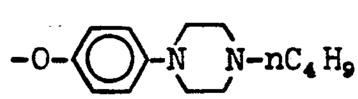 | |

| Verb. Nr. | Ar | A | n | $R_1$ | Q | Fp. [°C] |
|---|---|---|---|---|---|---|
| 165 | 4-$C_6H_4Cl$ | CH | 4 | H | 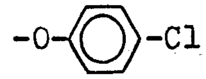 | 148-149 |
| 166 | 4-$C_6H_4Cl$ | CH | 4 | $CH_3$ | 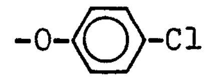 | Oel |
| 167 | 4-$C_6H_4Cl$ | CH | 4 | H | 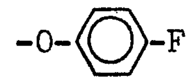 | |
| 168 | 4-$C_6H_4Cl$ | N | 4 | H | 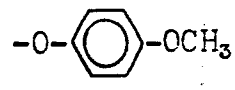 | |
| 169 | 4-$C_6H_4Cl$ | CH | 4 | H | 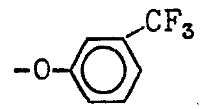 | |
| 170 | 4-$C_6H_4Cl$ | CH | 4 | H | 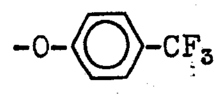 | |
| 171 | 4-$C_6H_4Cl$ | CH | 4 | H | 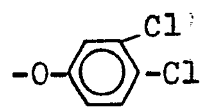 | |
| 172 | 4-$C_6H_4Cl$ | CH | 4 | H | 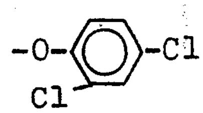 | |
| 173 | 4-$C_6H_4Cl$ | CH | 4 | H | 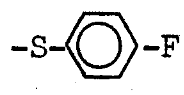 | |
| 174 | 4-$C_6H_4Cl$ | CH | 4 | H | 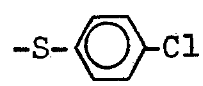 | 137-138 |
| 175 | 4-$C_6H_4Cl$ | CH | 4 | H | 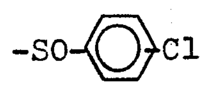 | Harz |
| 178 | 4-$C_6H_4Cl$ | CH | 4 | H | 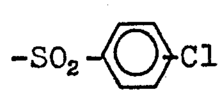 | 128-123 |

| Verb. Nr. | Ar | A | n | $R_1$ | Q | Fp. [°C] |
|---|---|---|---|---|---|---|
| 179 | $4-C_6H_4Cl$ | N | 4 | H | 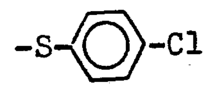 | 83- 84 |
| 180 | $4-C_6H_4Cl$ | N | 4 | H | 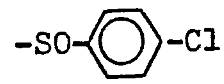 | 120-123 |
| 181 | $4-C_6H_4Cl$ | N | 4 | H | 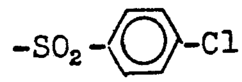 | 157-158 |
| 182 | $4-C_6H_4Cl$ | N | 4 | H | 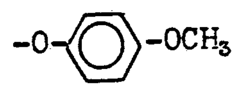 | |
| 183 | $4-C_6H_4Cl$ | CH | 4 | H | 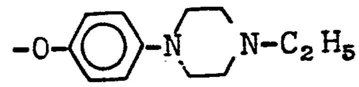 | |
| 184 | $4-C_6H_4Cl$ | CH | 4 | H | 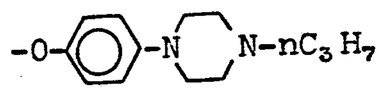 | |
| 185 | $4-C_6H_4Cl$ | CH | 4 | H | 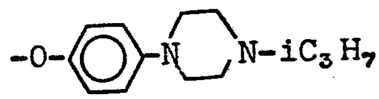 | |
| 186 | $4-C_6H_4Cl$ | CH | 4 | H | 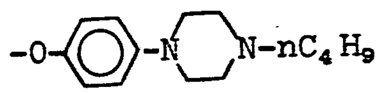 | |
| 187 | $2,4-C_6H_3Cl_2$ | CH | 4 | H | 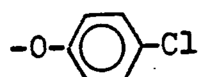 | |
| 188 | $2,4-C_6H_3Cl_2$ | CH | 4 | H | 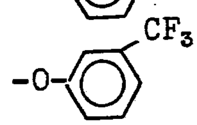 | |
| 189 | $2,4-C_6H_3Cl_2$ | CH | 4 | H | 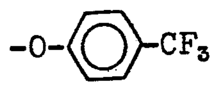 | |
| 190 | $2,4-C_6H_3Cl_2$ | CH | 4 | H | 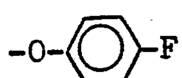 | |
| 191 | $2,4-C_6H_3Cl_2$ | CH | 4 | H | 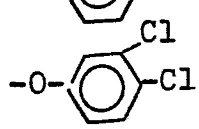 | |
| 192 | $2,4-C_6H_3Cl_2$ | CH | 4 | H | 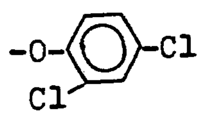 | |
| 193 | $2,4-C_6H_3Cl_2$ | CH | 4 | H | 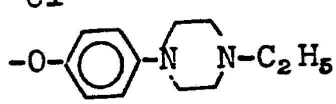 | |

| Verb. Nr. | Ar | A | n | $R_1$ | Q | Fp. [°C] |
|---|---|---|---|---|---|---|
| 194 | $2,4-C_6H_3Cl_2$ | CH | 4 | H | 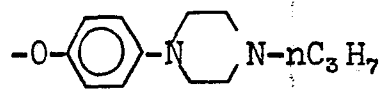 | |
| 195 | $2,4-C_6H_3Cl_2$ | CH | 4 | H | 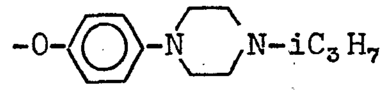 | |
| 196 | $2,4-C_6H_3Cl_2$ | CH | 4 | H | 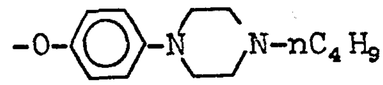 | |
| 197 | $2,4-C_6H_3Cl_2$ | N | 4 | H | 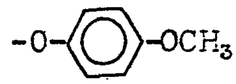 | |
| 198 | $2,4-C_6H_3Cl_2$ | CH | 4 | H | 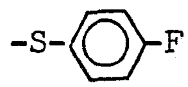 | |
| 199 | $2,4-C_6H_3Cl_2$ | CH | 4 | H | 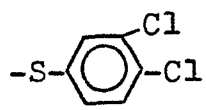 | |
| 200 | $4-C_6H_4Cl$ | CH | 5 | H | 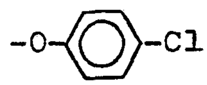 | 149-150 |
| 201 | $4-C_6H_4Cl$ | CH | 5 | H | 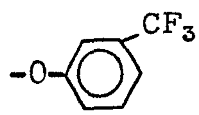 | 105-107 |
| 202 | $4-C_6H_4Cl$ | CH | 5 | H | 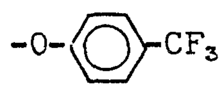 | 129-130 |
| 203 | $4-C_6H_4Cl$ | CH | 5 | H | 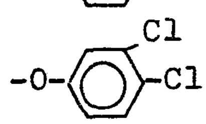 | 117-118 |
| 204 | $4-C_6H_4Cl$ | CH | 5 | H | 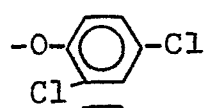 | |
| 205 | $4-C_6H_4Cl$ | CH | 5 | H | 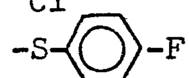 | |
| 206 | $4-C_6H_4Cl$ | CH | 5 | H | 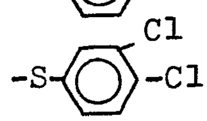 | |
| 207 | $4-C_6H_4Cl$ | N | 5 | H | 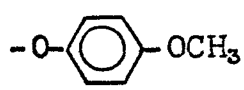 | |
| 208 | $4-C_6H_4Cl$ | CH | 5 | H | 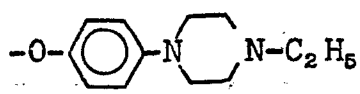 | |

| Verb. Nr. | Ar | A | n | $R_1$ | Q | Fp. [°C] |
|---|---|---|---|---|---|---|
| 209 | $4-C_6H_4Cl$ | CH | 5 | H | 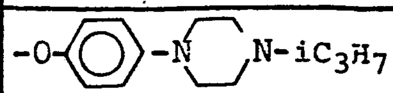 | |
| 210 | $4-C_6H_4Cl$ | CH | 5 | H | 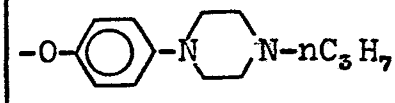 | |
| 212 | $4-C_6H_4Cl$ | CH | 5 | H | 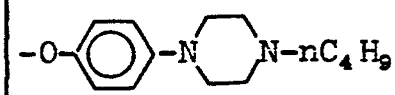 | |
| 213 | $2,4-C_6H_3Cl_2$ | CH | 5 | H | 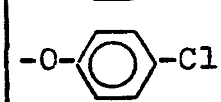 | |
| 214 | $2,4-C_6H_3Cl_2$ | CH | 5 | H | 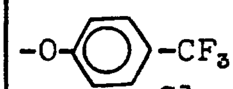 | |
| 215 | $2,4-C_6H_3Cl_2$ | CH | 5 | H | 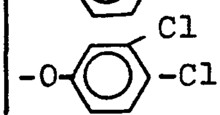 | |
| 216 | $2,4-C_6H_3Cl_2$ | CH | 5 | H | 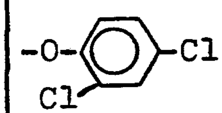 | |
| 217 | $2,4-C_6H_3Cl_2$ | CH | 5 | H | 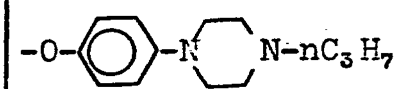 | |
| 218 | $2,4-C_6H_3Cl_2$ | CH | 5 | H | 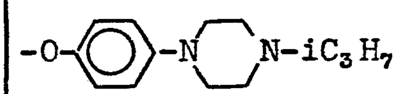 | |
| 219 | $2,4-C_6H_3Cl_2$ | CH | 5 | H | 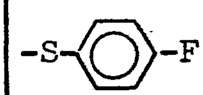 | |
| 220 | $2,4-C_6H_3Cl_2$ | N | 5 | H | 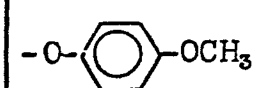 | |
| 221 | $2,4-C_6H_3Cl_2$ | CH | 5 | H | 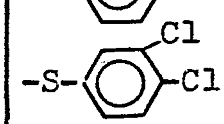 | |
| 222 | $2,4-C_6H_3Cl_2$ | CH | 6 | H | 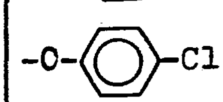 | |
| 223 | $2,4-C_6H_3Cl_2$ | CH | 6 | H | 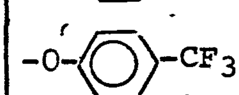 | |
| 224 | $2,4-C_6H_3Cl_2$ | CH | 6 | H | 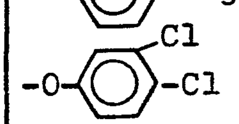 | |
| 225 | $2,4-C_6H_3Cl_2$ | CH | 6 | H | 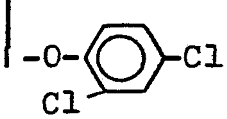 | |

| Verb. Nr. | Ar | A | n | $R_1$ | Q | Fp. |
|---|---|---|---|---|---|---|
| 226 | 2,4-$C_6H_3Cl_2$ | CH | 6 | H | 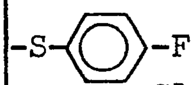 | |
| 227 | 2,4-$C_6H_3Cl_2$ | CH | 6 | H | 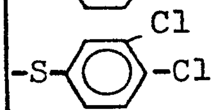 | |
| 228 | 2,4-$C_6H_3Cl_2$ | CH | 7 | H | 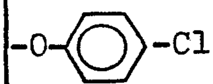 | |
| 229 | 2,4-$C_6H_3Cl_2$ | CH | 7 | H | 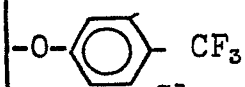 | |
| 230 | 2,4-$C_6H_3Cl_2$ | CH | 7 | H | 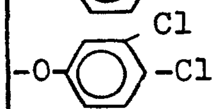 | |
| 231 | 2,4-$C_6H_3Cl_2$ | CH | 7 | H | 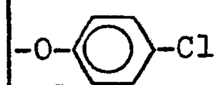 | |
| 232 | 2,4-$C_6H_3Cl_2$ | CH | 8 | H | 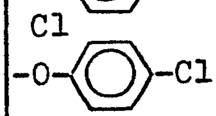 | |
| 233 | 2,4-$C_6H_3Cl_2$ | CH | 8 | H | 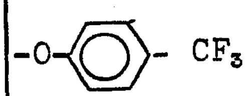 | |
| 234 | 2,4-$C_6H_3Cl_2$ | CH | 8 | H | 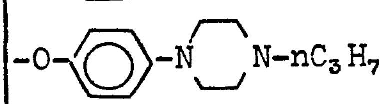 | |
| 235 | 2,4-$C_6H_3Cl_2$ | CH | 8 | H | 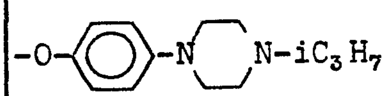 | |
| 236 | 2,4-$C_6H_3Cl_2$ | CH | 9 | H | 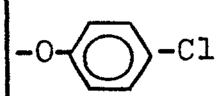 | |
| 237 | 2,4-$C_6H_3Cl_2$ | CH | 9 | H | 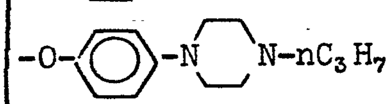 | |
| 238 | 2,4-$C_6H_3Cl_2$ | CH | 2 | H | 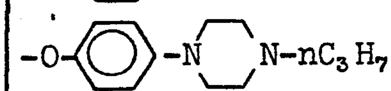 | |
| 239 | 2,4-$C_6H_3Cl_2$ | CH | 2 | H | 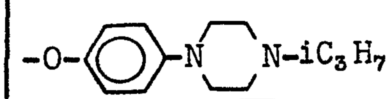 | |
| 240 | 4-$C_6H_4Cl$ | N | 3 | H | 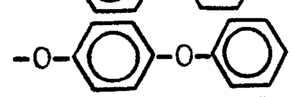 | 106-108 |
| 241 | 4-$C_6H_4Cl$ | N | 4 | H | 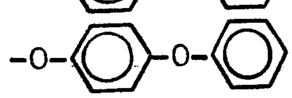 | |
| 242 | 4-$C_6H_4Cl$ | N | 5 | H | 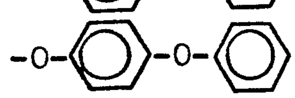 | |
| 243 | 4-$C_6H_4Cl$ | N | 6 | H | 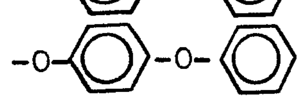 | |

| Verb. Nr. | Ar | A | n | $R^1$ | Q | Fp [°C] |
|---|---|---|---|---|---|---|
| 266 | $4-C_6H_4Cl$ | CH | 2 | H | 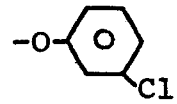 | 77-79° |
| 267 | $4-C_6H_4Cl$ | N | 2 | H | 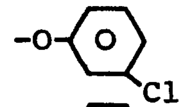 | Harz |
| 268 | $4-C_6H_4Cl$ | CH | 2 | H | 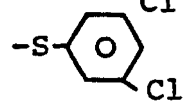 | Harz |
| 269 | $4-C_6H_4Cl$ | CH | 2 | H | 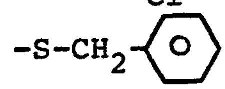 | 151-152° |
| 270 | $2,4-C_6H_3Cl_2$ | N | 3 | H | 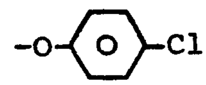 | 69-71° |
| 271 | $2,4-C_6H_3Cl_2$ | N | 3 | H | $-OCH_3$ | 138-139 |

Herstellung der Ausgangsprodukte der allgemeinen Formel (VII)

Beispiel 11

2-(4-Fluorphenyl)-1-(1H-imidazol-1-yl) -pentan-2,5-diol

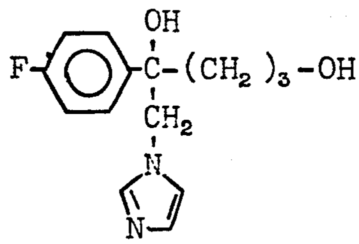

a) γ-(4-Chlorphenyl)-γ-(1 H-imidazol-1-ylmethyl)-γ-butyrolakton

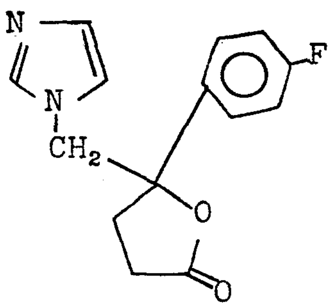

Eine aus 165,0 g Trimethylsulfoxoniumjodid und 21,0 g Natriumhydrid (80 %ige Dispersion in Mineralöl) in 700 ml trockenem Dimethylsulfoxid analog Beispiel 1 hergestellte Lösung von Dimethylsulfoxoniummethylid wird zu einer Lösung von 105,0 g 3-(4-Fluorbenzoyl)-propionsäure-methylester in 200 ml trockenem Dimethylsulfoxid während einer Stunde zugetropft. Man rührt zwei Stunden bei 50 °C, kühlt ab und gießt auf Eis. Man nimmt mit Methylenchlorid auf, schüttelt die organische Phase zweimal mit Wasser, trocknet und zieht das Lösungsmittel ab. Die rohe Oxiran-Zwischenstufe wird mit 100 g Imidazol 30 Minuten auf dem Dampfbad erhitzt, das Gemisch mit Wasser/Methylenchlorid aufgenommen, die

organische Phase mit Wasser gewaschen, getrocknet und eingeengt. Beim Verreiben des Rückstands mit Ethylacetat kristallisiert das Produkt aus.

Ausbeute: 45,0 g farblose Kristalle (35 % d.Th.)
Fp. 109 - 111 °C

Analyse:

$C_{14}H_{13}FN_2O_2$ MG 260,27

ber.: C 64,61 %; H 5,03 %; N 10,7 %
gef.: C 64,4 %; H 5,0 %; N 10,6 %

b) 2-(4-Fluorphenyl)-1-(1H-imidazol-1-yl)-pentan-2,5-diol

In einem Rundkolben wird eine Lösung von 1,5 g Lithium aluminiumhydrid in 50 ml trockenem Tetrahydrofuran vorgelegt und unter Rühren eine Lösung von 10,0 g des unter a) hergestellten Produkts in 100 ml trockenem Tetrahydrofuran zugetropft. Man rührt zwei Stunden bei 50 °C, kühlt ab und zersetzt vorsichtig mit Wasser. Nach Stehen über Nacht wird vom anorganischen Material abfiltriert und das Lösungsmittel abgezogen.
Beim Verreiben des öligen Rückstands mit Diethylether kristallisieren 8,8 g farblose Kristalle aus (87 % d.Th.)
Fp: 119°

Analyse:

$C_{14}H_{17}FN_2O_2$ MG 261, 28

ber.: C 63,62 %; H 6,48 %; N 10,60 %
gef.: C 63,8 %; H 6,6 %; N 10,4 %

Beispiel 12

2-(4-Chlorphenyl)-1-(1H-imidazol-1-yl)-butan-2,4-diol

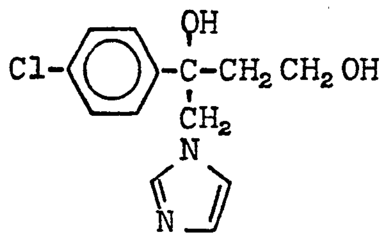

a) 3-(4-Chlorphenyl)-4-(1H-imidazol-1-yl)-3-hydroxy-buttersäureethylester

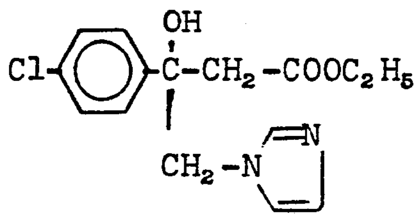

25 ml einer 1 M-Lösung von Lithium-bis(trimethylsilyl)-amid in Tetrahydrofuran werden auf - 78 °C gekühlt und 2.4 ml Ethylacetat zugetropft. Nach 15-minütigem Rühren wird bei - 78 °C eine Lösung von 5.5 g (4-Chlorphenyl)-(1H-imidazol-1-ylmethyl)-keton in 70 ml trockenem Tetrahydrofuran zugetropft. Man läßt 30 Minuten bei - 78 °C nachrühren, gießt auf Eis und extrahiert mit Methylenchlorid. Die organische Phase wird getrocknet und eingeengt. Nach Chromatographie an Kieselgel erhält man 3.3 g farblose Kristalle (43% d.Th.).

b) 2-(4-Chlorphenyl)-1-(1H-imidazol-1-yl)-butan-2,4-diol

3.9 g des unter a) erhaltenen Produkts werden analog Beispiel 11b) mit 500 mg Lithiumaluminiumhydrid reduziert.

Das Rohprodukt wird aus Ethylacetat umkristallisiert. Man erhält 2.3 g farblose Kristalle (68 % d,Th.)
Fp 127 - 129°

| | | | | | |
|---|---|---|---|---|---|
| Analyse | ber. | C | 58,54 | gef. C | 58.3 |
| $C_{13}H_{15}ClNO_2O_2$ | | H | 5,67 | H | 5,4 |
| MG 266,73 | | N | 10,50 | N | 10,3 |

Beispiel 13

2-(4-Chlorphenyl)-1-(1H-imidazol-1-yl)-heptan-2,7-diol

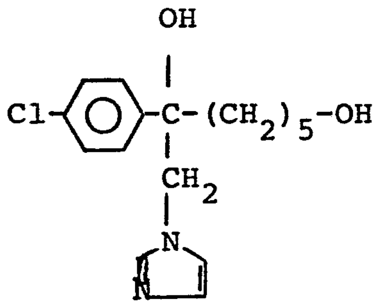

a) 6-(4-Chlorphenyl)-7-(1H-imidazol-1-yl)-6-hydroxy-önanthsäure-methylester

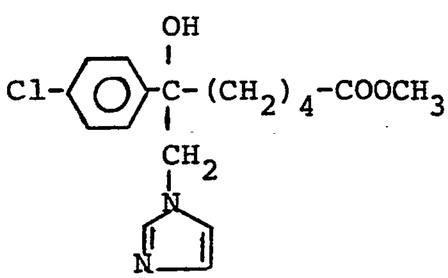

Analog Beispiel 1 wird aus 105,6 g Trimethylsulfoxoniumjodid, 13,2 g Natriumhydrid (80 %ige Dispersion in Mineralöl) und 101,9 g 5-(4-Chlorbenzoyl)-valeriansäuremethylester (vgl. J. Amer. Chem. Soc. 70, 3197 (1948)) die Oxiranzwischenstufe hergestellt und diese ohne Reinigung mit 100 g Imidazol 30 Minunten auf dem Dampfbad erhitzt. Aufarbeitung mit Wasser/ Methylenchlorid und Reinigung des Rohprodukt durch Chromatographie an Kieselgel mit Ethylacetat/ Methanol im Verhältnis 9 : 1 ergibt 71.0 g farbloses Öl, das beim

Verreiben mit Ethylacetat kristallisiert (52.7 % d.Th.).
Fp 98 - 99°

Analyse:

$C_{17}H_{21}ClN_2O_3$ MG 336,82

| | | | | | |
|---|---|---|---|---|---|
| ber.: | C | 60,62 | gef.: | C | 60.6 |
| | H | 6.28 | | H | 6.2 |
| | N | 8.32 | | N | 8.2 |

b) 2-(4-Chlorphenyl)-1-(1H-imidazol-1-yl)-heptan-2,7-diol

60 g des unter a) hergestellten Esters werden in 200 ml trockenem Tetrahydrofuran gelöst zu einer Lösung von 6.0 g Lithiumaluminiumhydrid in 200 ml trockenem Tetrahydrofuran unter Kühlung zugetropft. Man rührt 2 Stunden bei 50°, kühlt ab und zersetzt vorsichtig mit Wasser. Nach Stehen über Nacht wird vom anorganischen Material abgesaugt und das Lösungsmittel eingeengt. Beim Verreiben des öligen Rückstands mit Ethylacetat kristallisieren 32 g farbloses Produkt aus (58,1 % d. Th.).
Fp. 102 - 103°

Analyse:

$C_{16}H_{21}ClN_2O_2$ MG 308,81

| | | | | | |
|---|---|---|---|---|---|
| ber.: | C | 62.23 | gef.: | C | 61.5 |
| | H | 6.85 | | H | 6.8 |
| | N | 9.07 | | N | 8.6 |

Die folgende Tabelle 2 enthält weitere Verbindungen der allgemeinen Formel (VII), die analog den Beispielen 11, 12 und 13 hergestellt werden können.

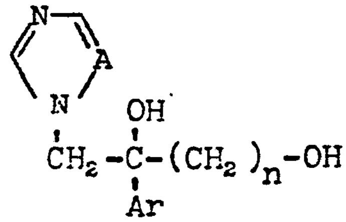 (VII)

| Verb. Nr. | A | n | Ar | Fp. [°C] |
|---|---|---|---|---|
| 244 | CH | 3 | 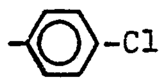 | 127-128 (Nitrat) |
| 245 | N | 3 | 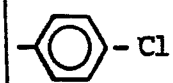 | 127-128 |
| 246 | CH | 3 | 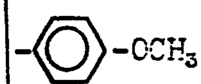 | Harz |
| 247 | CH | 3 | 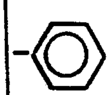 | 121-122 |
| 248 | CH | 3 | 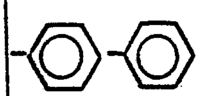 | 157 |
| 250 | CH | 4 | 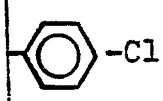 | 124-125 |
| 251 | N | 3 | 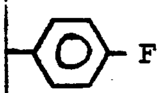 | 119-120 |
| 252 | CH | 3 | 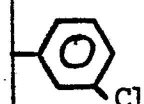 | Harz |
| 252 | CH | 3 | 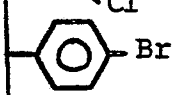 | 118-120 |
| 254 | CH | 3 | 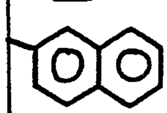 | 176-177 |
| 255 | CH | 3 | 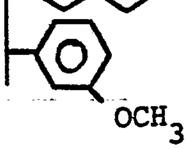 | 122-123 |

| Verb. Nr. | A | n | Ar | Fp [°C] |
|---|---|---|---|---|
| 256 | CH | 2 | 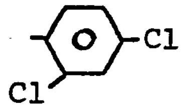 | 144 - 145 |
| 257 | CH | 3 | 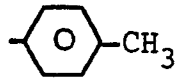 | 151 - 153 |
| 258 | CH | 3 | 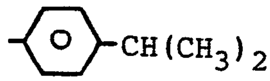 | Harz |
| 259 | CH | 3 | 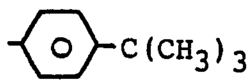 | Harz |
| 260 | CH | 3 | 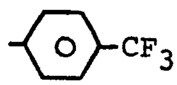 | 151 - 152 |
| 261 | CH | 3 | 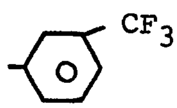 | 102 - 104 |
| 262 | CH | 3 | 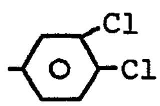 | 132 - 133 |
| 263 | CH | 3 | 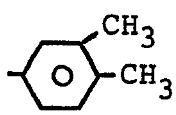 | Harz |
| 264 | CH | 3 | 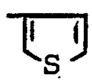 | 105 - 108 |
| 265 | CH | 3 | 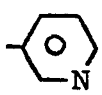 | Harz |

Herstellung der Ausgangsverbindungen der allgemeinen Formel (IV)

Beispiel 14

1-(4-Fluorbenzoyl)-3-(3,4-dichlorphenoxy)-propan

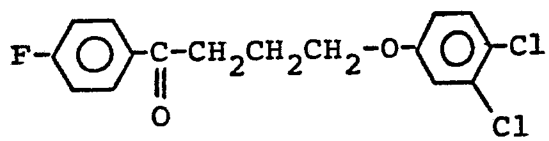

Zu einer Lösung von 59.9 g 3,4-Dichlorphenol in 200 ml trockenem Dimethylformamid gibt man unter Kühlung portionsweise 9,9 g Natriumhydrid (80 %ige Dispersion in Mineralöl). Nach Ende der Wasserstoffentwicklung gibt man 73,4 g (2-(4-Fluorphenyl)-2-(3-chlorpropyl)-1,3-dioxolan (hergestellt nach CA 63 9959 d (1965)) zu und rührt 6 Stunden bei 80°. Man zieht das Lösungsmittel am Vakuum ab und erhitzt den öligen Rückstand unter kräftigem Rühren mit einem Gemisch aus Wasser/konzentrierter Salzsäure im Verhältnis 2:1 4 Stunden unter Rückfluß. Nach Abkühlen wird mit Methylenchlorid aufgenommen und die organische Phase mit 2 n Natronlauge und Wasser gewaschen, getrocknet und eingeengt. Umkristallisation aus Ethylacetat ergibt 50,0 g farbloses Produkt. (46.3 % d.Th.) Fp. 96 - 97°.

Beispiel 15

1-(4-Chlorbenzoyl)-4-(4-chlorphenylthio)-butan

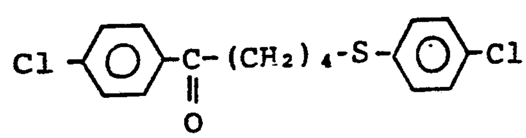

Zu einer aus 8,2 g Natrium in 500 ml Ethanol hergestellten Natriumethylatlösung gibt man 56,3 g 4-Chlorthiophenol und 74,9 g ω-Chlor-4-chlorvalerophenon (hergestellt aus

Chlorbenzol und 5-Chlorvaleriansäurechlorid analog Industrie Chimique Belge 9, 1073 (1960) und erhitzt 5 Stunden unter Rückfluß. Man filtriert heiß ab, kühlt ab und saugt das ausgefallene Produkt ab. Durch Einengen der Mutterlauge wird weiteres Produkt gewonnen.
Ausbeute: 87,5 g farblose Kristalle (79,6 % d.Th.)
Fp. 95 - 96°.

Beispiel 16

1-(4-Chlorbenzoyl)-3-[4-(4-acetylpiperazino)-phenoxy]-propan

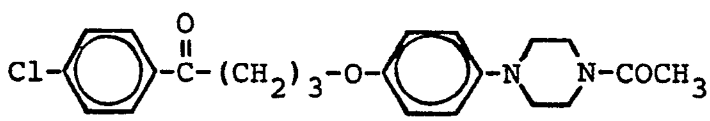

a.) 1-(4-Chlorbenzoyl)-3-(4-piperazinophenoxy)-propan

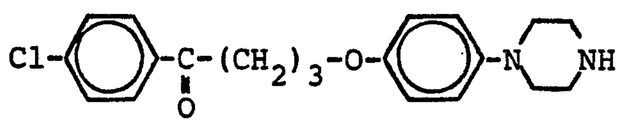

Zu einer Lösung von 36,6 g 1-(4-Hydroxyphenyl)-piperazin in 200 ml trockenem Dimethylformamid gibt man portionsweise 6,0 g Natriumhydrid (80 %ige Dispersion in Mineralöl) und rührt bei 50° bis zum Ende der Wasserstoffentwicklung. Nach der Zugabe von 52 g 2-(4-Chlorphenyl)-2-[3-chlorpropyl]-1,3-dioxolan (hergestellt nach CA 63 9959 d (1965)) und rührt 8 Stunden bei 80°. Man zieht das Lösungsmittel am Ölpumpenvakuum ab und erhitzt den öligen Rückstand 6 Stunden mit einem Gemisch aus 125 ml konz. Salzsäure und 250 ml Wasser unter Rückfluß. Man verdünnt noch in der Hitze mit 500 ml Wasser, läßt abkühlen und schüttelt die Wasserphase zweimal mit Diethylether aus. Durch Zugabe von Soda wird die Wasserphase alkalisch gestellt und das ausgeschiedene Öl mit Methylenchlorid extrahiert. Das

nach dem Trocknen und Abziehen des Methylenchlorids zurückbleibende Öl wird mit Ethylacetat verrieben. Es kristallisieren 30,6 g farblose Kristalle aus (43 % d.Th.).

Fp. 145°

b) 1-(4-Chlorbenzoyl)-3-[4-(4-acetylpiperazino)-phenoxy]-propan

Das unter a) erhaltene Produkt wird 6 Stunden mit überschüssigem Acetanhydrid unter Rückfluß erhitzt. Abziehen des überschüssigen Acetanhydrids und Umkristallisieren des Rückstands aus Ethanol ergibt 30.7 g farblose Schuppen (90 % d.Th.).

Fp. 144°

Patentansprüche

1. Azolylalkyl-Derivate der Formel (I)

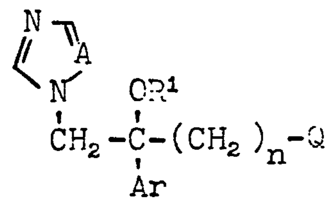

(I)

in welcher

A die CH-Gruppe oder ein Stickstoffatom bedeutet,

n die Zahlen 2 - 12 bedeutet,

Ar eine gegebenenfalls einen, zwei oder drei Substituenten tragende Phenylgruppe bedeutet, wobei die Substituenten gleich oder verschieden sein können und jeweils ein Halogenatom, eine $C_1$-$C_6$-alkyl- oder $C_1$-$C_6$-alkoxygruppe oder eine Trifluormethylgruppe bedeuten, oder auch eine gegebenenfalls mit einem Halogenatom oder einer $C_1$-$C_6$-alkyl- oder $C_1$-$C_6$-alkoxygruppe substituierte Biphenyl-, Naphthyl-, Phenoxy=phenyl-, Phenyl-$C_1$-$C_6$-alkyl-, Phenyl-$C_1$-$C_6$-alkoxyphenyl- oder Thienylgruppe bedeutet,

$R^1$ ein Wasserstoffatom, eine $C_1$-$C_{12}$-Alkyl-, $C_2$-$C_{10}$-Alkenyl-, $C_2$-$C_6$-Alkinyl- oder eine Benzylgruppe bedeutet, die am Benzolring gegebenenfalls ein oder zwei Substituenten tragen kann, wobei diese gleich oder verschieden sein können und jeweils ein Halogenatom oder eine $C_1$-$C_6$-alkyl-, $C_1$-$C_6$-alkoxy- oder Trifluormethylgruppe bedeuten,

Q entweder

a) einen Rest der Formel (II)

$$-S(O)_x-R^2 \qquad (II)$$

bedeutet,
wobei x für O, 1 und 2 steht und
$R^2$ eine $C_1$-$C_{12}$-Alkyl-, $C_3$-$C_8$-Cycloalkyl-, $C_3$-$C_8$-Cycloalkyl-$C_1$-$C_{12}$-alkyl-, $C_2$-$C_{10}$-Alkenyl-, Pyridyl-, 2-Furylmethyl-, Naphthyl-, Halogennaphthyl-, $C_1$-$C_6$-alkylnaphthyl-, Naphthylmethyl-, Phenyl- oder Benzylgruppe bedeutet wobei bei den zwei letztgenannten Gruppen die Benzolringe gegebenenfalls ein oder zwei Stubstituenten tragen können, die gleich oder verschieden sein können und jeweils Halogen, $C_1$-$C_6$-alkyl, $C_1$-$C_6$-alkoxy, Trifluormethyl, Phenyl, Nitro, Amino oder Aminoacetyl bedeuten können,
oder

b) einen Rest-$OR^3$ bedeutet, wobei
$R^3$ eine $C_1$-$C_{12}$-Alkyl-, $C_3$-$C_8$-Cycloalkyl-, $C_3$-$C_8$-Cycloalkyl-$C_1$-$C_{12}$-alkyl-, $C_2$-$C_{10}$-Alkenyl-, $C_2$-$C_6$-Alkinyl, $C_1$-$C_6$-alkoxy-$C_1$-$C_6$-alkyl-, $C_1$-$C_6$-alkylthio-$C_1$-$C_6$-alkyl-, Phenylthio-$C_1$-$C_6$-alkyl-, Phenyloxy-$C_1$-$C_6$-alkyl-, Thienylmethyl-, Furylmethyl-, Pyridyl-, Naphthyl-, Halogennaphthyl, $C_1$-$C_6$-alkylnaphthyl-, $C_1$-$C_6$-alkoxynaphthyl-, Naphthylmethyl-, Phenyl-, Phenyl-$C_1$-$C_6$-alkyl- oder Phenyl-$C_2$-$C_6$-alkenylgruppe bedeutet, wobei bei den drei letztgenannten Gruppen die Phenylgruppe gegebenenfalls einen, zwei oder drei Substituenten tragen kann, die gleich oder verschieden sein können und jeweils ein Halogenatom oder eine $C_1$-$C_6$-alkyl-, Halogen-$C_1$-$C_6$-alkyl, $C_1$-$C_6$-alkoxy, Halogen-$C_1$-$C_6$-alkoxy-, Nitro-, Cyano-, Amino-,

Aminoacetyl-, $C_1$-$C_6$-alkylamino-, Di-$C_1$-$C_6$-alkylamino-, Phenyl-, Phenyloxy-, Phenyl-$C_1$-$C_6$-alkyl- oder Phenyl-$C_1$-$C_6$-alkoxygruppe bedeuten, mit der Maßgabe, daß nur einer der Substituenten eine Phenyl-, Phenyloxy-, Phenyl-$C_1$-$C_6$-alkyl- oder Phenyl-$C_1$-$C_6$-alkoxygruppe darstellt, die ihrerseits gegebenenfalls einen oder zwei Substituenten tragen können, die gleich oder verschieden sein können und jeweils eine $C_1$-$C_6$-alkyl-, $C_1$-$C_6$-alkoxy-, Nitro-, Cyano- oder Trifluormethylgruppe oder ein Halogenatom bedeuten, und $R^3$ weiterhin einen Rest der Formel (III) bedeutet

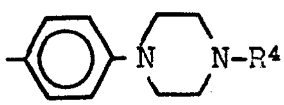 (III)

wobei $R^4$ eine $C_1$-$C_6$-alkyl- oder $C_1$-$C_6$-alkylcarbonylgruppe bedeutet, mit der Maßgabe, daß für diejenigen Verbindungen für die $R^3$ einen Rest der Formel (III) bedeutet, n nicht die Zahl 6 bedeutet, sowie ihre Salze mit Säuren, Metallsalzkomplexe und ihre Stereoisomeren.

2. Verfahren zur Herstellung von Verbindungen der Formel (I a) aus Anspruch 1, für die Ar, A, n und Q die in Anspruch 1 angegebenen Bedeutungen haben und $R^{1'}$ Wasserstoff bedeutet,

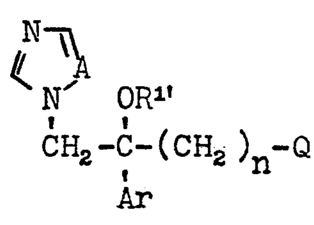 (I a)

dadurch gekennzeichnet, daß man eine Verbindung der Formel (IV)

$$Ar-\underset{\overset{\|}{O}}{C}-(CH_2)_n-Q \quad (IV)$$

für die Ar, Q und n die in Anspruch 1 angegebenen Bedeutungen haben,

mit einem Schwefelylid der Formel (A)

$$(CH_3)_2S(O)_ZCH_2 \quad (A)$$

worin Z null oder eins bedeutet, in Gegenwart eines Verdünnungsmittels zu einer Verbindung der allgemeinen Formel (V)

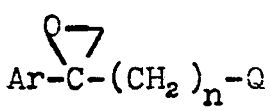
(V)

umsetzt, für die Ar, n und Q die in Anspruch 1 angegebenen Bedeutungen haben, und anschließend eine Verbindung der Formel (V) mit einer Verbindung der Formel (VI) umsetzt,

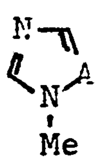
(VI)

in der Me ein Wasserstoffatom oder ein Alkali- oder Erdalkalimetallatom bedeutet, und A die in Anspruch 1 angegebenen Bedeutungen hat, und gegebenenfalls

a) eine Verbindung der Formel (I), in der Q $-S-R^2$ aus Anspruch 1 bedeutet, oxidiert, oder

b) eine Verbindung der Formel (I), in der Q

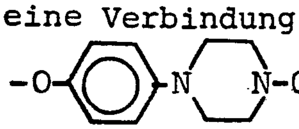$-CO-C_1-C_6$-alkyl bedeutet,

mittels eines komplexen Metall-Hydrids reduziert.

3. Verfahren zur Herstellung von Verbindungen der Formel (I b) aus Anspruch 1

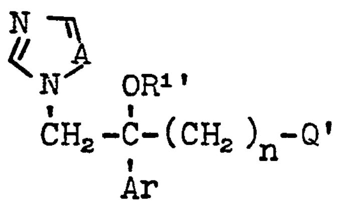

(I b)

worin A, Ar und n die in Anspruch 1 angegebenen Bedeutungen haben, $R^{1'}$ Wasserstoff bedeutet und Q' einen Rest OPh oder $S(O)_xPh$ bedeutet, worin x für O, 1 oder 2 steht und Ph eine Naphthyl-, Halogennaphthyl-, $C_1$-$C_6$-alkylnaphthyl, $C_1$-$C_6$- alkoxynaphthyl- oder eine Phenylgruppe bedeutet, die gegebenenfalls einen, zwei oder drei Substituenten tragen kann, die gleich oder verschieden sein können und jeweils ein Halogenatom oder eine $C_1$-$C_6$- alkyl-, Halogen-$C_1$-$C_6$-alkyl-, $C_1$-$C_6$-alkoxy-, Halogen-$C_1$-$C_6$-alkoxy-, Nitro-, Cyano-, Amino-, Aminoacetyl-, $C_1$-$C_6$- alkylamino-, Di-$C_1$-$C_6$alkyl=amino-, Phenyl-, Phenyloxy-, Phenyl-$C_1$-$C_6$-alkyl- oder Phenyl-$C_1$-$C_6$ alkoxygruppe bedeuten, mit der Maßgabe, daß nur einer der Substituenten eine Phenyl-, Phenyloxy-, Phenyl-$C_1$-$C_6$- alkyl- oder Phenyl-$C_1$-$C_6$-alkoxygruppe darstellt, die ihrerseits gegebenenfalls einen oder zwei

Substituenten tragen kann die gleich oder verschieden sein können und jeweils eine $C_1$-$C_6$-alkyl-, $C_1$-$C_6$-alkoxy-, Nitro-, Cyano- oder Trifluormethylgruppe oder ein Halogenatom bedeuten,

und für den Fall, daß Q' einen Rest OPh bedeutet, Ph weiterhin einen Rest der Formel (III)

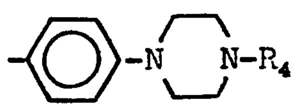 (III)

bedeutet,
wobei $R^4$ eine $C_1$-$C_6$-alkyl- oder $C_1$-$C_6$-alkylcarbonylgruppe bedeutet, mit der Maßgabe, daß für diejenigen Verbindungen für die $R^3$ einen Rest der Formel (III) bedeutet, n nicht die Zahl 6 bedeutet, dadurch gekennzeichnet, daß man eine Verbindung der allgemeinen Formel (VII)

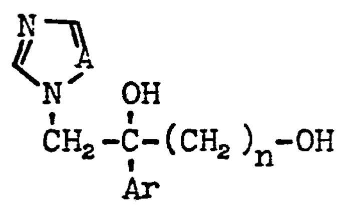 (VII)

in der Ar, A und n die in Anspruch 1 angegebenen Bedeutungen haben, mit einer Verbindung der Formel (VIII),

H-XPh (VIII)

in der X Sauerstoff oder Schwefel bedeutet und Ph die oben angegebenen Bedeutungen hat, in Gegenwart von Triphenylphosphin und eines Azodicarbonsäuredialkylesters

$C_1$-$C_{12}$-Alkyl-OOC-N=N-COO-$C_1$-$C_{12}$-Alkyl

umsetzt, und die so erhaltenen Verbindungen der Formel (I b) gegebenenfalls gemäß Anspruch 2 am Schwefel oxidiert oder an der Amidgruppe reduziert.

4. Verfahren zur Herstellung von Verbindungen der Formel (I a) aus Anspruch 2, dadurch gekennzeichnet, daß man eine Verbindung der Formel (VII),

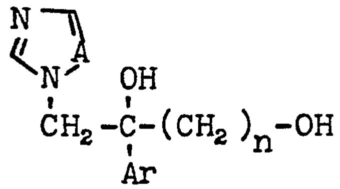 (VII)

in der A, Ar und n die in Anspruch 1 angegebenen Bedeutungen haben, mit Hilfe eines Halogenierungsmittels oder eines Sulfonylierungsmittels in eine Verbindung der allgemeinen Formel (IX) überführt,

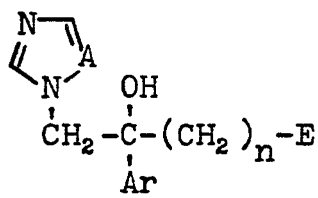 (IX)

in der A, Ar und n die in Anspruch 1 angegebenen Bedeutungen haben und E eine reaktive Abgangsgruppe bedeutet, vorzugsweise ein Halogenatom oder eine Alkyl-, Fluoralkyl- oder Arylsulfonyloxygruppe,

und anschließend eine Verbindung der Formel (IX) mit einer Verbindung der Formel (X) umsetzt,

Met-Q" (X)

in der Met ein Alkali- oder Erdalkali-Metallatom bedeutet und Q" einen Rest der Formel $-SR^2$ oder $-OR^3$, worin $R^2$ und $R^3$ die in Anspruch 1

angegebenen Bedeutungen haben, und gegebenenfalls die so erhaltenen Verbindungen der Formel (I a) gemäß Anspruch 2 oxidiert oder reduziert.

5. Verfahren zur Herstellung von Verbindungen der Formel (Ic)

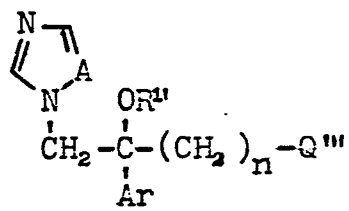

(I c)

in der A, Ar und n die in Anspruch 1 angegebenen Bedeutungen haben und Q"' einen Rest $OR^{3'}$ bedeutet, worin $R^{3'}$ eine $C_1$-$C_{12}$-Alkyl-, $C_3$-$C_8$-Cycloalkyl-, $C_3$-$C_8$-Cycloalkyl-$C_1$-$C_{12}$-alkyl-, $C_2$-$C_{10}$-Alkenyl-, $C_2$-$C_6$-Alkinyl-, $C_1$-$C_6$-alkoxy-$C_1$-$C_6$ alkyl-, $C_1$-$C_6$-alkylthio-$C_1$-$C_6$-alkyl-, Phenylthio-$C_1$-$C_6$-alkyl-, Phenyl=oxy-$C_1$-$C_6$-alkyl-, Thienylmethyl-, Furylmethyl-, Naphthylmethyl-, Phenyl-$C_1$-$C_6$-alkyl- oder Phenylnieder=alkenylgruppe bedeutet, wobei bei den zwei letztgenannten Gruppen die Phenylgruppe gegebenenfalls bis zu drei Substituenten tragen kann und diese Substituenten gleich oder verschieden sein können und jeweils ein Halogenatom oder eine $C_1$-$C_6$- alkyl-, Halogen-$C_1$-$C_6$-alkyl-, $C_1$-$C_6$- alkoxy-, Halogen-$C_1$-$C_6$-alkoxy-, Nitro-, Cyano-, Amino-, Aminoacetyl-, $C_1$-$C_6$- alkyl-amino-, Di-$C_1$-$C_6$-alkylamino-, Phenyl-, Phenyloxy-, Phenyl-$C_1$-$C_6$-alkyl- oder Phenyl-$C_1$-$C_6$- alkoxygruppe bedeuten können, mit der Einschränkung, daß nur einer

der Substituenten eine Phenyl-, Phenyloxy-, Phenyl-$C_1$-$C_6$- alkyl- oder Phenyl-$C_1$-$C_6$-alkoxygruppe darstellt, die ihrerseits gegebenenfalls bis zu zwei Substituenten tragen können, wobei diese Substituenten gleich oder verschieden sein können und jeweils eine Niederalkyl-, Niederalkoxy-, Nitro-, Cyano- oder Trifluormethylgruppe oder ein Halogenatom bedeuten,

dadurch gekennzeichnet, daß man eine Verbindung der allgemeinen Formel (VII) aus Anspruch 3 in Gegenwart einer starken Base mit einer Verbindung der allgemeinen Formel (XI) umsetzt,

$R^{3'}$-E (XI)

in der $R^{3'}$ die oben angegebenen Bedeutungen hat und E eine reaktive Abgangsgruppe darstellt.

6. Verfahren zur Herstellung von Verbindungen der Formel (I d)

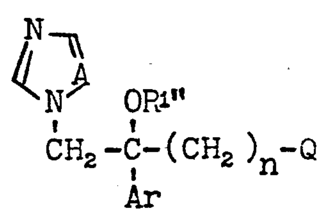

(I d)

in der A, Ar, n und Q die in Anspruch 1 angegebenen Bedeutungen haben und $R^{1''}$ eine Alkyl-, Alkenyl-, Alkinyl- oder gegebenenfalls substituierte Benzylgruppe nach Anspruch 1 bedeutet, dadurch gekennzeichnet, daß man eine Verbindung der allgemeinen Formel (I a) aus An-

spruch 2 mit Hilfe einer starken Base in ein Metallalkoholat überführt und dieses mit einer Verbindung der allgemeinen Formel $ER^{1''}$ umsetzt, in der $R^{1''}$ die oben angegebenen Bedeutungen hat und E eine reaktive Abgangsgruppe ist, und die so erhaltenen Verbindungen der Formel (I d) gegebenenfalls gemäß Anspruch 2 am Schwefel oxidiert oder an einer Amidgruppe reduziert.

7. Verbindungen der Formel (VII a)

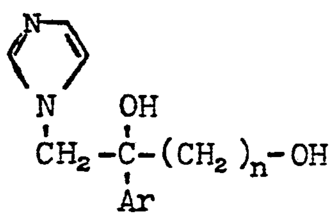

(VII a )

für die n und Ar die Anspruch 1 angegebenen Bedeutungen haben, sowie ihre Salze mit Säuren und ihre Stereoisomeren.

8. Verfahren zur Herstellung von Verbindungen der Formel (VII a')

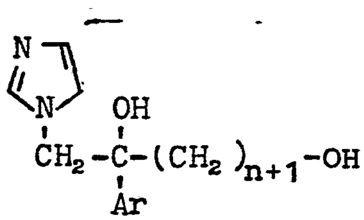

(VII a)

für die A, n und Ar die in Anspruch 1 angegebenen Bedeutungen haben, dadurch gekennzeichnet, daß man eine Verbindung der allgemeinen Formel (XII)

$$Ar-\underset{\overset{\|}{O}}{C}-(CH_2)_n-COOAlk \qquad (XII)$$

in der Ar und n die oben angegebenen Bedeutungen haben und Alk eine $C_1$-$C_{12}$-Alkylgruppe bedeutet,

mit einem Schwefelylid der Formel (A)

$$(CH_3)_2S(O)_zCH_2 \quad (A)$$

worin z null oder eins bedeutet, in Gegenwart eines Verdünnungsmittels zu einer Verbindung der Formel (XIII) umsetzt,

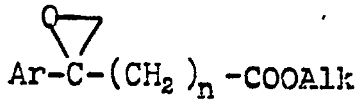

(XIII)

für die Ar und n die oben angegebenen Bedeutungen haben und Alk eine Alkylgruppe bedeutet,

anschließend eine Verbindung der Formel (XIII) mit einer Verbindung der Formel (VI')

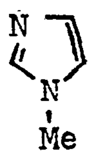

(VI')

umsetzt, worin Me die zur Formel (VI) angegebene Bedeutung hat, und hierbei eine Verbindung der Formel (XIV) oder (XV) erhält,

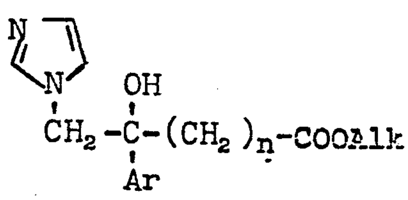

(XIV)

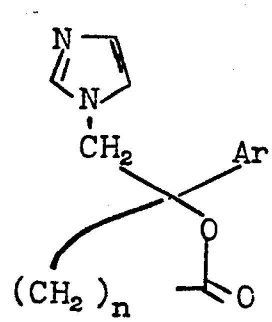

(XV)

für die Ar und n die oben angegebenen Bedeutungen haben und Alk eine Alkylgruppe bedeutet, und anschließend eine Verbindung der Formel (XIV) oder (XV) mit einem komplexen Metallhydrid zu einer Verbindung der Formel (VII a') reduziert.

9. Verfahren zur Herstellung von Verbindungen der Formel (VII a")

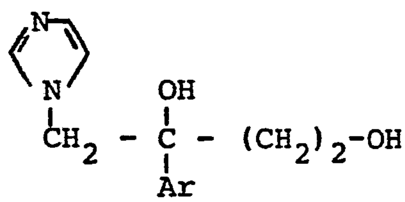

(VII a")

worin Ar die in Anspruch 1 angegebenen Bedeutungen hat, dadurch gekennzeichnet, daß man eine Verbindung der Formel (XVI),

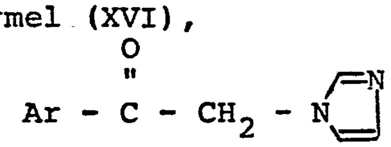

(XVI)

für die Ar die in Anspruch 1 angegebenen Bedeutungen hat, mit einem Essigsäureester $CH_3$-COOAlk, für den Alk eine Alkylgruppe bedeutet, in Gegenwart einer starken Base zu einer Verbindung der Formel (XVII) umsetzt,

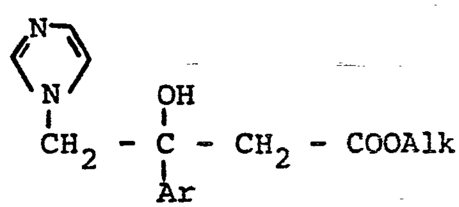

(XVII)

worin Ar und Alk die oben angegebenen Bedeutungen haben, und anschließend eine Verbindung der Formel (XVII) mit einem komplexen Metallhydrid zu einer Verbindung der Formel (VII a") reduziert.

10. Verbindungen der Formel (VII b)

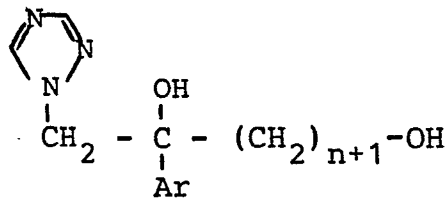

(VII b)

für die Ar und n die in Anspruch 1 angegebenen Bedeutungen haben.

11. Verfahren zur Herstellung von Verbindungen der Formel (VII b),

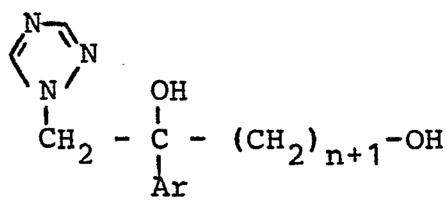

(VII b)

für die Ar und n die in Anspruch 1 angegebenen Bedeutungen haben, dadurch gekennzeichnet, daß man eine Verbindung der Formel (XII)

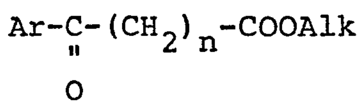

(XII)

in der Ar und n die oben angegebenen Bedeutungen haben und Alk eine $C_1$-$C_{12}$-Alkylgruppe bedeutet, mit einem Schwefelylid der allgemeinen Formel (A)

$$(CH_3)_2S(O)_zCH_2 \quad (A) \qquad z = 0,1$$

in Gegenwart eines Verdünnungsmittels zu einer Verbindung der Formel (XIII) umsetzt,

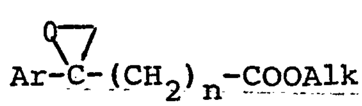

(XIII)

für die Ar und n die oben angegebenen Bedeutungen haben und Alk eine Alkylgruppe bedeutet,

0150036
HOE 84/F 012

anschließend eine Verbindung der Formel (XIII) mit einer Verbindung der Formel (VI")

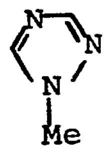

(VI")

umsetzt, worin Me die zur Formel (VI) angegebene Bedeutung hat, und hierbei eine Verbindung der Formel (XVIII) oder (XIX) erhält,

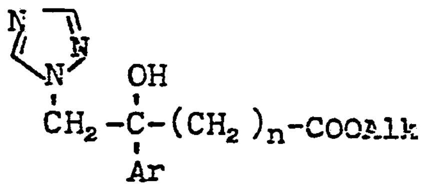

(XVIII)

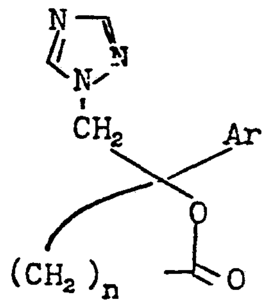

(XIX)

für die Ar und n die oben angegebenen Bedeutungen haben und Alk eine Alkylgruppe bedeutet, und anschließend eine Verbindung der Formel (XVIII) oder (XIX) mit einem komplexen Metallhydrid zu einer Verbindung der Formel (VII b) reduziert.

12. Arzneimittel, dadurch gekennzeichnet, daß es eine Verbindung gemäß Anspruch 1 enthält oder aus ihr besteht.

13. Verfahren zur Herstellung einer pharmazeutischen Zubereitung nach Anspruch 12, dadurch gekennzeichnet, daß man eine Verbindung der Formel (I) oder eines ihrer Salze, gegebenenfalls zusammen mit einem üblichen pharmazeutischen Trägerstoff und/oder Hilfsstoff, in eine für pharmazeutische Zwecke geeignete Darreichungsform bringt.

14. Verwendung einer Verbindung gemäß Anspruch 1 zur Bekämpfung von Mykosen, Protozoen und grampositiver und gramnegativer Bakterien.

15. Pharmazeutische Zubereitung, dadurch gekennzeichnet, daß sie eine Verbindung der allgemeinen Formel (VII a) oder eine ihrer Salze oder Stereoisomeren enthält oder aus ihr besteht.

16. Verfahren zur Herstellung einer pharmazeutischen Zubereitung nach Anspruch 15, dadurch gekennzeichnet, daß man eine Verbindung der allgemeinen Formel (VII a) oder eine ihrer Salze oder Stereoisomeren, gegebenenfalls zusammen mit einem üblichen pharmazeutischen Trägerstoff und/oder Hilfsstoff, in eine für pharmazeutische Zwecke geeignete Darreichungsform bringt.

17. Verwendung einer Verbindung der allgemeinen Formel (VII a) oder eine ihrer Salze oder Stereoisomeren zur Behandlung depressiver Zustände.

18. Schädlingsbekämpfungsmittel, gekennzeichnet durch einen Gehalt an einer Verbindung der allgemeinen Formel (I) in Anspruch 1.

19. Verfahren zur Bekämpfung oder Verhinderung eines Befalls von Kulturpflanzen durch phytopathogene Pilze, dadurch gekennzeichnet, daß man eine Verbindung der Formel (I) gemäß Anspruch 1 auf die Pflanze bzw. auf durch Pilzbefall bedrohte Flächen, Pflanzen oder Saatgüter einwirken läßt.

20. Verfahren zur Herstellung eines fungiziden Mittels gemäß Anspruch 18, dadurch gekennzeichnet, daß man eine Verbindung der Formel (I) gemäß Anspruch 1 mit festen oder flüssigen Trägerstoffen sowie gegebenenfalls mit einem oder mehreren oberflächenaktiven Mitteln mischt.

Patentansprüche Österreich:

1. Verfahren zur Herstellung von Azolylalkyl-Derivaten der Formel (I)

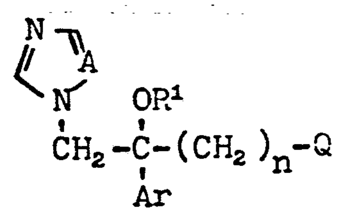

(I)

in welcher

A die CH-Gruppe oder ein Stickstoffatom bedeutet,

n die Zahlen 2 - 12 bedeutet,

Ar eine gegebenenfalls einen, zwei oder drei Substituenten tragende Phenylgruppe bedeutet, wobei die Substituenten gleich oder verschieden sein können und jeweils ein Halogenatom, eine $C_1$-$C_6$-alkyl- oder $C_1$-$C_6$-alkoxygruppe oder eine Trifluormethylgruppe bedeuten,
oder auch eine gegebenenfalls mit einem Halogenatom oder einer $C_1$-$C_6$-alkyl- oder $C_1$-$C_6$-alkoxygruppe substituierte Biphenyl-, Naphthyl-, Phenoxy=phenyl-, Phenyl-$C_1$-$C_6$-alkyl-, Phenyl-$C_1$-$C_6$-alkoxyphenyl- oder Thienylgruppe bedeutet,

$R^1$ ein Wasserstoffatom, eine $C_1$-$C_{12}$-Alkyl-, $C_2$-$C_{10}$-Alkenyl-, $C_2$-$C_6$-Alkinyl- oder eine Benzylgruppe bedeutet, die am Benzolring gegebenenfalls ein oder zwei Substituenten tragen kann, wobei diese gleich oder verschieden sein können und jeweils ein Halogenatom oder eine $C_1$-$C_6$-alkyl-, $C_1$-$C_6$-alkoxy- oder Trifluormethylgruppe bedeuten,

Q entweder

a) einen Rest der Formel (II)

$$-S(O)_x-R^2 \quad (II)$$

bedeutet,
wobei x für O, 1 und 2 steht und
$R^2$ eine $C_1$-$C_{12}$-Alkyl-, $C_3$-$C_8$-Cycloalkyl-, $C_3$-$C_8$-Cycloalkyl-$C_1$-$C_{12}$-alkyl-, $C_2$-$C_{10}$-Alkenyl-, Pyridyl-, 2-Furylmethyl-, Naphthyl-, Halogennaphthyl-, $C_1$-$C_6$-alkylnaphthyl-, Naphthylmethyl-, Phenyl- oder Benzylgruppe bedeutet wobei bei den zwei letztgenannten Gruppen die Benzolringe gegebenenfalls ein oder zwei Stubstituenten tragen können, die gleich oder verschieden sein können und jeweils Halogen, $C_1$-$C_6$-alkyl, $C_1$-$C_6$-alkoxy, Trifluormethyl, Phenyl, Nitro, Amino oder Aminoacetyl bedeuten können,
oder

b) einen Rest-$OR^3$ bedeutet, wobei
$R^3$ eine $C_1$-$C_{12}$-Alkyl-, $C_3$-$C_8$-Cycloalkyl-, $C_3$-$C_8$-Cycloalkyl-$C_1$-$C_{12}$-alkyl-, $C_2$-$C_{10}$-Alkenyl-, $C_2$-$C_6$-Alkinyl, $C_1$-$C_6$-alkoxy-$C_1$-$C_6$-alkyl-, $C_1$-$C_6$-alkylthio-$C_1$-$C_6$-alkyl-, Phenylthio-$C_1$-$C_6$-alkyl-, Phenyloxy-$C_1$-$C_6$-alkyl-, Thienylmethyl-, Furylmethyl-, Pyridyl-, Naphthyl-, Halogennaphthyl, $C_1$-$C_6$-alkylnaphthyl-, $C_1$-$C_6$-alkoxynaphthyl-, Naphthylmethyl-, Phenyl-, Phenyl-$C_1$-$C_6$-alkyl- oder Phenyl-$C_2$-$C_6$-alkenylgruppe bedeutet, wobei bei den drei letztgenannten Gruppen die Phenylgruppe gegebenenfalls einen, zwei oder drei Substituenten tragen kann, die gleich oder verschieden sein können und jeweils ein Halogenatom oder eine $C_1$-$C_6$-alkyl-, Halogen-$C_1$-$C_6$-alkyl, $C_1$-$C_6$-alkoxy, Halogen-$C_1$-$C_6$-alkoxy-, Nitro-, Cyano-, Amino-,

Aminoacetyl-, $C_1$-$C_6$-alkylamino-, Di-$C_1$-$C_6$-alkylamino-, Phenyl-, Phenyloxy-, Phenyl-$C_1$-$C_6$-alkyl- oder Phenyl=$C_1$-$C_6$-alkoxygruppe bedeuten, mit der Maßgabe, daß nur einer der Substituenten eine Phenyl-, Phenyloxy-, Phenyl-$C_1$-$C_6$-alkyl- oder Phenyl-$C_1$-$C_6$-alkoxygruppe darstellt, die ihrerseits gegebenenfalls einen oder zwei Substituenten tragen können, die gleich oder verschieden sein können und jeweils eine $C_1$-$C_6$-alkyl-, $C_1$-$C_6$-alkoxy-, Nitro-, Cyano- oder Trifluormethylgruppe oder ein Halogenatom bedeuten, und $R^3$ weiterhin einen Rest der Formel (III) bedeutet

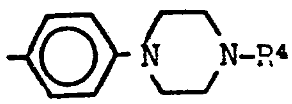 (III)

wobei $R^4$ eine $C_1$-$C_6$-alkyl- oder $C_1$-$C_6$-alkylcarbonylgruppe bedeutet, mit der Maßgabe, daß für diejenigen Verbindungen für die $R^3$ einen Rest der Formel (III) bedeutet, n nicht die Zahl 6 bedeutet, sowie ihre Salze mit Säuren, Metallsalzkomplexe und ihre Stereoisomeren, dadurch gekennzeichnet, daß man eine Verbindung der Formel (IV)

$$Ar-\underset{\overset{\|}{O}}{C}-(CH_2)_n-Q \quad (IV)$$

für die Ar, Q und n die angegebenen Bedeutungen haben,

mit einem Schwefelylid der Formel (A)

$$(CH_3)_2S(O)_ZCH_2 \quad (A)$$

worin Z null oder eins bedeutet, in Gegenwart eines Verdünnungsmittels zu einer Verbindung der allgemeinen Formel (V)

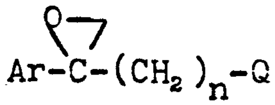 (V)

umsetzt,
für die Ar, n und Q die angegebenen Bedeutungen haben,
und anschließend eine Verbindung der Formel (V) mit einer Verbindung der Formel (VI) umsetzt,

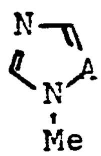 (VI)

in der Me ein Wasserstoffatom oder ein Alkali- oder Erdalkalimetallatom bedeutet, und A die angegebenen Bedeutungen hat,
und gegebenenfalls

a) eine Verbindung der Formel (I), in der Q $-S-R^2$ bedeutet, oxidiert, oder

b) eine Verbindung der Formel (I), in der Q 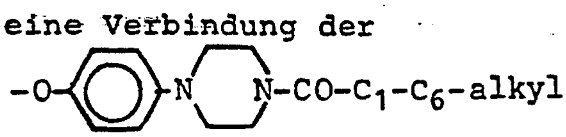 bedeutet,

mittels eines komplexen Metall-Hydrids reduziert,
und gegebenenfalls die so erhaltenen Verbindungen I, für die $R^1$ Wasserstoff bedeutet, mit Hilfe einer starken Base in ein Metallalkoholat überführt

und dieses mit einer Verbindung der allgemeinen Formel $ER^{1''}$ umsetzt, in der $R^{1''}$ eine Alkyl-, Alkenyl-, Alkinyl- oder Benzylgruppe, wie oben für $R^1$ definiert, und E eine reaktive Abgangsgruppe ist.

2. Verfahren nach Anspruch 1 zur Herstellung von Verbindungen der Formel (I b)

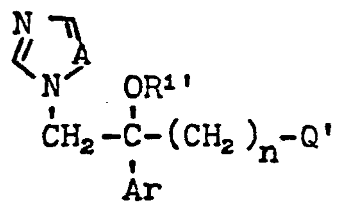 (I b)

worin A, Ar und n die in Anspruch 1 angegebenen Bedeutungen haben, $R^{1'}$ Wasserstoff bedeutet und Q' einen Rest OPh oder $S(O)_xPh$ bedeutet, worin x für O, 1 oder 2 steht und Ph eine Naphthyl-, Halogennaphthyl-, $C_1$-$C_6$-alkylnaphthyl, $C_1$-$C_6$- alkoxynaphthyl- oder eine Phenylgruppe bedeutet, die gegebenenfalls einen, zwei oder drei Substituenten tragen kann, die gleich oder verschieden sein können und jeweils ein Halogenatom oder eine $C_1$-$C_6$- alkyl-, Halogen-$C_1$-$C_6$-alkyl-, $C_1$-$C_6$-alkoxy-, Halogen-$C_1$-$C_6$-alkoxy-, Nitro-, Cyano-, Amino-, Aminoacetyl-, $C_1$-$C_6$- alkylamino-, Di-$C_1$-$C_6$alkyl=amino-, Phenyl-, Phenyloxy-, Phenyl-$C_1$-$C_6$-alkyl- oder Phenyl-$C_1$-$C_6$alkoxygruppe bedeuten, mit der Maßgabe, daß nur einer der Substituenten eine Phenyl-, Phenyloxy-, Phenyl-$C_1$-$C_6$- alkyl- oder Phenyl-$C_1$-$C_6$-alkoxygruppe darstellt, die ihrerseits gegebenenfalls einen oder zwei Substituenten tragen kann die gleich oder verschieden sein können und jeweils eine $C_1$-$C_6$- alkyl-, $C_1$-$C_6$-alkoxy-, Nitro-, Cyano- oder Trifluormethylgruppe oder ein Halogenatom bedeuten,

und für den Fall, daß Q' einen Rest OPh bedeutet, Ph weiterhin einen Rest der Formel (III)

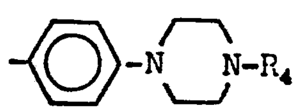 (III)

bedeutet,
wobei $R^4$ eine $C_1$-$C_6$- alkyl- oder $C_1$-$C_6$- alkylcarbonylgruppe bedeutet, mit der Maßgabe, daß für diejenigen Verbindungen für die $R^3$ einen Rest der Formel (III) bedeutet, n nicht die Zahl 6 bedeutet,

dadurch gekennzeichnet, daß man eine Verbindung der allgemeinen Formel (VII)

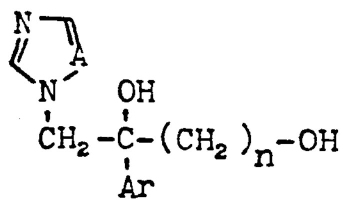 (VII)

in der Ar, A und n die in Anspruch 1 angegebenen Bedeutungen haben, mit einer Verbindung der Formel (VIII),

H-XPh (VIII)

in der X Sauerstoff oder Schwefel bedeutet und Ph die oben angegebenen Bedeutungen hat, in Gegenwart von Triphenylphosphin und eines Azodicarbonsäuredialkylesters

$C_1$-$C_{12}$-Alkyl-OOC-N=N-COO-$C_1$-$C_{12}$-Alkyl

umsetzt, und die so erhaltenen Verbindungen der Formel (I b) gegebenenfalls am Schwefel oxidiert oder an der Amidgruppe reduziert.

3. Verfahren nach Anspruch 1 zur Herstellung von Verbindungen der Formel (I a)

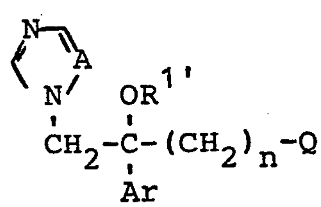 (I a),

in der Ar, A, n und Q die angegebenen Bedeutungen haben, und $R^{1'}$ Wasserstoff bedeutet, dadurch gekennzeichnet, daß man eine Verbindung der Formel (VII),

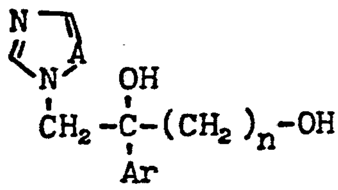 (VII)

in der A, Ar und n die angegebenen Bedeutungen haben, mit Hilfe eines Halogenierungsmittels oder eines Sulfonylierungsmittels in eine Verbindung der allgemeinen Formel (IX) überführt,

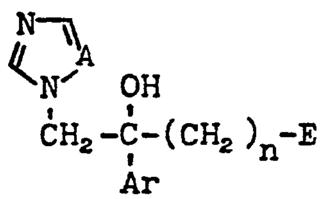 (IX)

in der A, Ar und n die angegebenen Bedeutungen haben und E eine reaktive Abgangsgruppe bedeutet, vorzugsweise ein Halogenatom oder eine Alkyl-, Fluoralkyl- oder Arylsulfonyloxygruppe,

und anschließend eine Verbindung der Formel (IX) mit einer Verbindung der Formel (X) umsetzt,

Met-Q" (X)

in der Met ein Alkali- oder Erdalkali-Metallatom bedeutet und Q" einen Rest der Formel $-SR^2$ oder $-OR^3$, worin $R^2$ und $R^3$ die angegebenen Bedeutungen haben, und gegebenenfalls die so erhaltenen Verbindungen der Formel (I a) oxidiert oder reduziert.

4. Verfahren nach Anspruch 1 zur Herstellung von Verbindungen der Formel (I c)

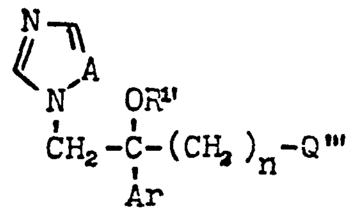

(I c)

in der A, Ar und n die in Anspruch 1 angegebenen Bedeutungen haben und Q"' einen Rest $OR^{3'}$ bedeutet, worin $R^{3'}$ eine $C_1$-$C_{12}$-Alkyl-, $C_3$-$C_8$-Cycloalkyl-, $C_3$-$C_8$-Cycloalkyl-$C_1$-$C_{12}$-alkyl-, $C_2$-$C_{10}$-Alkenyl-, $C_2$-$C_6$-Alkinyl-, $C_1$-$C_6$-alkoxy-$C_1$-$C_6$ alkyl-, $C_1$-$C_6$-alkylthio-$C_1$-$C_6$-alkyl-, Phenylthio-$C_1$-$C_6$-alkyl-, Phenyl=oxy-$C_1$-$C_6$-alkyl-, Thienylmethyl-, Furylmethyl-, Naphthylmethyl-, Phenyl-$C_1$-$C_6$-alkyl- oder Phenylnieder=alkenylgruppe bedeutet, wobei bei den zwei letztgenannten Gruppen die Phenylgruppe gegebenenfalls bis zu drei Substituenten tragen kann und diese Substituenten gleich oder verschieden sein können und jeweils ein Halogenatom oder eine $C_1$-$C_6$- alkyl-, Halogen-$C_1$-$C_6$-alkyl-, $C_1$-$C_6$-alkoxy-, Halogen-$C_1$-$C_6$-alkoxy-, Nitro-, Cyano-, Amino-, Aminoacetyl-, $C_1$-$C_6$-alkylamino-, Di-$C_1$-$C_6$-alkylamino-, Phenyl-, Phenyloxy-, Phenyl-$C_1$-$C_6$-alkyl- oder Phenyl-$C_1$-$C_6$- alkoxygruppe bedeuten können, mit der Einschränkung, daß nur einer der Substituenten eine Phenyl-, Phenyloxy-, Phenyl-$C_1$-$C_6$- alkyl- oder Phenyl-$C_1$-$C_6$- alkoxygruppe darstellt, die ihrerseits gegebenenfalls bis zu zwei Substituenten tragen können, wobei diese Substituenten gleich oder verschieden sein können und jeweils eine Niederalkyl-, Niederalkoxy-, Nitro-, Cyano- oder Trifluormethylgruppe oder ein Halogenatom bedeuten,

dadurch gekennzeichnet, daß man eine Verbindung der allgemeinen Formel (VII) aus Anspruch 2 in Gegenwart einer starken Base mit einer Verbindung der allgemeinen

Formel (XI) umsetzt,

$$R^{3'}-E \quad (XI)$$

in der $R^{3'}$ die oben angegebenen Bedeutungen hat und E eine reaktive Abgangsgruppe darstellt.

5. Verfahren zur Herstellung von Verbindungen der Formel (VII a')

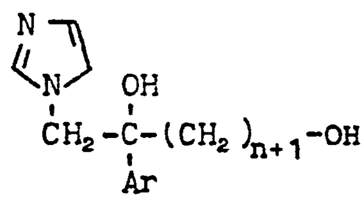

(VII a')

für die A, n und Ar die in Anspruch 1 angegebenen Bedeutungen haben, dadurch gekennzeichnet, daß man eine Verbindung der allgemeinen Formel (XII)

$$Ar-C(=O)-(CH_2)_n-COOAlk \quad (XII)$$

in der Ar und n die oben angegebenen Bedeutungen haben und Alk eine $C_1$-$C_{12}$-Alkylgruppe bedeutet,

mit einem Schwefelylid der Formel (A)

$$(CH_3)_2S(O)_zCH_2 \quad (A)$$

worin z null oder eins bedeutet, in Gegenwart eines Verdünnungsmittels zu einer Verbindung der Formel (XIII) umsetzt,

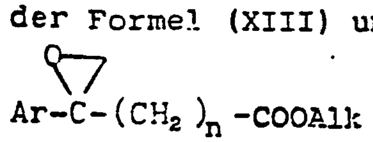

(XIII)

für die Ar und n die oben angegebenen Bedeutungen haben und Alk eine Alkylgruppe bedeutet,

 

anschließend eine Verbindung der Formel (XIII) mit einer Verbindung der Formel (VI')

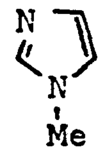 (VI')

umsetzt, worin Me die zur Formel (VI) angegebene Bedeutung hat, und hierbei eine Verbindung der Formel (XIV) oder (XV) erhält,

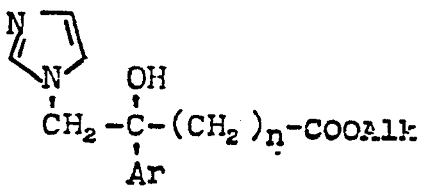

(XIV)

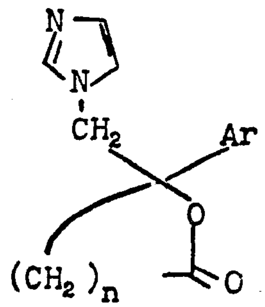 (XV)

für die Ar und n die oben angegebenen Bedeutungen haben und Alk eine Alkylgruppe bedeutet, und anschließend eine Verbindung der Formel (XIV) oder (XV) mit einem komplexen Metallhydrid zu einer Verbindung der Formel (VII a') reduziert.

6. Verfahren zur Herstellung von Verbindungen der Formel (VII a")

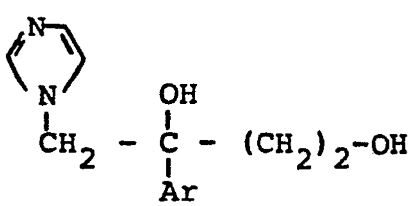 (VII a"),

worin Ar die in Anspruch 1 angegebenen Bedeutungen hat,

dadurch gekennzeichnet, daß man eine Verbindung der Formel (XVI),

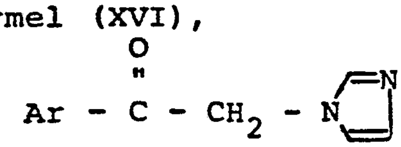 (XVI),

für die Ar die in Anspruch 1 angegebenen Bedeutungen hat, mit einem Essigsäureester $CH_3$-COOAlk, für den Alk eine Alkylgruppe bedeutet, in Gegenwart einer starken Base zu einer Verbindung der Formel (XVII) umsetzt,

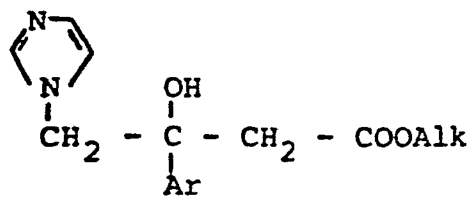 (XVII),

worin Ar und Alk die oben angegebenen Bedeutungen haben, und anschließend eine Verbindung der Formel (XVII) mit einem komplexen Metallhydrid zu einer Verbindung der Formel (VII a") reduziert.

7. Verfahren zur Herstellung von Verbindungen der Formel (VII b),

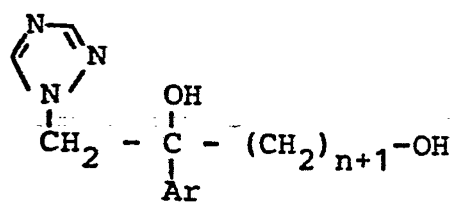 (VII b)

für die Ar und n die in Anspruch 1 angegebenen Bedeutungen haben, dadurch gekennzeichnet, daß man eine Verbindung der Formel (XII)

$$Ar-\underset{\underset{O}{\|}}{C}-(CH_2)_n-COOAlk \qquad (XII),$$

in der Ar und n die oben angegebenen Bedeutungen haben und Alk eine $C_1$-$C_{12}$-Alkylgruppe bedeutet, mit einem

Schwefelylid der allgemeinen Formel (A)

$(CH_3)_2S(O)_zCH_2$ (A) $z = 0;1$

in Gegenwart eines Verdünnungsmittels zu einer Verbindung der Formel (XIII) umsetzt,

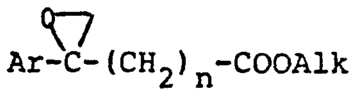

(XIII),

für die Ar und n die oben angegebenen Bedeutungen haben und Alk eine Alkylgruppe bedeutet,

anschließend eine Verbindung der Formel (XIII) mit einer Verbindung der Formel (VI")

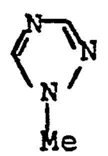

(VI")

umsetzt, worin Me die zur Formel (VI) angegebene Bedeutung hat, und hierbei eine Verbindung der Formel . (XVIII) oder (XIX) erhält,

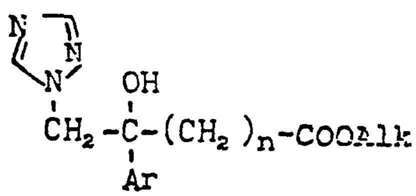

(XVIII)

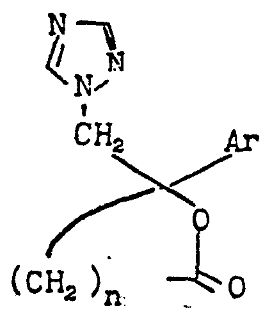

(XIX),

für die Ar und n die oben angegebenen Bedeutungen haben und Alk eine Alkylgruppe bedeutet,

und anschließend eine Verbindung der Formel (XVIII) oder (XIX) mit einem komplexen Metallhydrid zu einer Verbindung der Formel (VII b) reduziert.

8. Verfahren zur Herstellung einer pharmazeutischen Zubereitung, dadurch gekennzeichnet, daß man eine Verbindung der Formel (I) nach Anspruch 1 oder eines ihrer Salze, gegebenenfalls zusammen mit einem üblichen pharmazeutischen Trägerstoff und/oder Hilfsstoff, in eine für pharmazeutische Zwecke geeignete Darreichungsform bringt.

9. Verfahren zur Herstellung einer pharmazeutischen Zubereitung, dadurch gekennzeichnet, daß man eine Verbindung der allgemeinen Formel (VII a' oder VII a") hergestellt nach Ansprüchen 5 oder 6 oder eines ihrer Salze oder Stereoisomeren, gegebenenfalls zusammen mit einem üblichen pharmazeutischen Trägerstoff und/oder Hilfsstoff, in eine für pharmazeutische Zwecke geeignete Darreichungsform bringt.

**0150036**
Nummer der Anmeldung

Europäisches Patentamt

## EUROPÄISCHER TEILRECHERCHENBERICHT,
der nach Regel 45 des Europäischen Patentübereinkommens für das weitere Verfahren als europäischer Recherchenbericht gilt

EP 85100362.4

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.) 4 |
|---|---|---|---|
| D,A | EP - A1 - 0 054 974 (SUMITOMO CHEMICAL COMPANY)<br>* Ansprüche 1,26,27 *<br>-- | 1,18-20 | C 07 D 233/60<br>C 07 D 405/12<br>C 07 D 405/14<br>C 07 D 249/08<br>C 07 D 401/12<br>C 07 D 401/14<br>C 07 D 409/06<br>C 07 D 409/12<br>C 07 D 409/14<br>A 61 K 31/41<br>A 61 K 31/415<br>A 61 K 31/495<br>A 01 N 43/653<br>A 01 N 43/50 |
| D,A | EP - A1 - 0 072 623 (PFIZER CORPORATION)<br>* Ansprüche 1,6; Seite 7, Zeilen 20-27; Seite 8, Zeilen 1-8 *<br>-- | 1,12,15, 16 | |
| A | EP - A2/A3 - 0 090 993 (BAYER AG)<br>* Ansprüche 1,9,10 *<br>-- | 1,2,12, 15,16 | |
| A | EP - A1 - 0 095 828 (PFIZER LIMITED)<br>* Anspruch 1; Seite 5, Zeilen 1-3 *<br>-- | 1,2,12, 15,16 | RECHERCHIERTE SACHGEBIETE (Int. Cl.) 4<br>C 07 D 233/00<br>C 07 D 405/00<br>C 07 D 249/00<br>C 07 D 401/00<br>C 07 D 409/00<br>A 01 N 43/00 |

### UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-13, 15-16, 18-20
Unvollständig recherchierte Patentansprüche: –
Nicht recherchierte Patentansprüche: 14, 17
Grund für die Beschränkung der Recherche:

Verfahren zur therapeutischen Behandlung des menschlichen oder tierischen Körpers (Art. 52(4) EPÜ)

| Recherchenort | Abschlußdatum der Recherche | Prüfer |
|---|---|---|
| WIEN | 25-04-1985 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

0150036

Nummer der Anmeldung

Europäisches Patentamt

# EUROPÄISCHER TEILRECHERCHENBERICHT

EP 85100362.4

| EINSCHLÄGIGE DOKUMENTE | | | KLASSIFIKATION DER ANMELDUNG (Int. Cl.4) |
| --- | --- | --- | --- |
| Kategorie | Kennzeichnung des Dokuments mit Angabe, soweit erforderlich, der maßgeblichen Teile | betrifft Anspruch | |
| A | EP - A1 - 0 091 398 (CIBA-GEIGY AG)<br>* Ansprüche 1,23-26 *<br>-- | 1,2,18-20 | |
| A | DE - A1 - 2 736 122 (BASF AG)<br>* Anspruch 1 *<br>-- | 1,18-20 | |
| A | EP - A2/A3 - 0 038 109 (JANSSEN PHARMACEUTICA N.V.)<br>* Anspruch 1 *<br>---- | 1,18-20 | |
| | | | RECHERCHIERTE SACHGEBIETE (Int. Cl.4) |

EPA Form 1505.3 06.78